# EUROPÄISCHE PATENTSCHRIFT

(11) **EP 4 423 014 B1**
(45) Veröffentlichungstag und Bekanntmachung des Hinweises auf die Patenterteilung: **03.12.2025**
(21) Anmeldenummer: 22813105.8
(22) Anmeldetag: 26.10.2022
(51) Int. Cl.: C01C 1/242, C01C 3/02, C07C 231/06, C08F 220/14, C07C 67/18

(54) **OPTIMIERTES VERFAHREN ZUR HERSTELLUNG UND ISOLIERUNG VON BLAUSÄURE SOWIE VERFAHREN ZU DEREN UMSETZUNG ZU METHACRYLSÄURE (MAS) UND/ODER ALKYLMETHACRYLAT**
OPTIMIZED METHOD FOR THE PREPARATION AND ISOLATION OF HYDROCYANIC ACID AND METHOD FOR ITS CONVERSION TO METHACRYLIC ACID (MAS) AND / OR ALKYL METHACRYLATE
PROCÉDÉ OPTIMISÉ DE PRODUCTION ET D'ISOLATION DE L'ACIDE CYANHYDRIQUE, AINSI QUE SON PROCÉDÉ DE RÉACTION EN ACIDE MÉTHACRYLIQUE (AMS) ET/OU EN MÉTHACRYLATE D'ALKYLE

(30) Priorität: 29.10.2021 EP 21205535
(43) Veröffentlichungstag der Anmeldung: 04.09.2024
(73) Patentinhaber: Röhm GmbH, 64295 Darmstadt (DE)
(72) Erfinder: KRILL, Steffen, 64367 Mühltal (DE); DONG, Luyan, 63743 Aschaffenburg (DE); KLASOVSKY, Florian, 45721 Haltern am See (DE); HEIDEL, Sven, 64293 Darmstadt (DE)
(74) Vertreter: Röhm Patent Association
(86) Internationale Anmeldenummer: PCT/EP2022/079899
(87) Internationale Veröffentlichungsnummer: WO 2023/072998

(56) Entgegenhaltungen:
- WO-A1-2014/099568
- US-A- 2 656 251
- US-A- 5 087 737
- US-A1- 2016 194 210

## Beschreibung

Die vorliegende Erfindung betrifft ein verbessertes Verfahren zur Herstellung und Isolierung von Blausäure, wobei das Verfahren die Umsetzung von Methan, Ammoniak und optional Sauerstoff umfasst, wobei C2-C10 Nitril-Nebenprodukte, wie Acetonitril, Acrylnitril und Propionitril, in der nachfolgenden Aufarbeitung (Isolierung von Blausäure) durch eine optimierte Stoffstromführung in geeigneter Weise verteilt und effektiv aus dem Verfahren ausgeschleust werden. Somit können aus Sicherheits- und Qualitätsaspekten kritisch zu bewertende Polymer-Ablagerungen in der Anlage vermindert werden. Die Isolierung der Blausäure umfasst eine erste Absorption von nicht umgesetztem Ammoniak mit einem sauren, wässrigen Absorptionsmittel; eine zweite Absorption von HCN mit einem wässrigen Absorptionsmittel, das Erwärmen und die Destillation der wässrige HCN-haltigen Lösung.

Weiterhin betrifft die Erfindung ein Verfahren zur Herstellung von Methacrylsäure (MAS) und/oder Alkylmethacrylaten, insbesondere Methylmethacrylat (MMA), in einem ACH-Sulfo-Prozess unter Verwendung erfindungsgemäß hergestellter Blausäure.

### Stand der Technik

Es sind mehrere kommerziellen Verfahren bekannt, um im industriellen Maßstab Alkylmethacrylate, insbesondere Methylmethacrylat (MMA), herzustellen. Ein weltweit verbreitetes, kommerzielles Verfahren basiert auf Aceton als Grundstoff; die technologische Plattform hierzu wird meist als C3-Prozess oder ACH-Sulfo-Prozess bezeichnet. Hierbei wird Aceton mit Blausäure (Cyanwasserstoff, HCN) zum zentralen Zwischenprodukt Acetoncyanhydrin (ACH) umgesetzt. Dieses Zwischenprodukt wird isoliert und kann für die folgenden Prozessschritte zur Herstellung von Methacrylsäure (MAS) und Methylmethacrylat (MMA) eingesetzt werden. Ein solches Verfahren ist beispielweise in US 5,087,737 A beschrieben, wobei im letzten Schritt das als Zwischenprodukt erhaltene Methacrylsäureamid nicht mit Alkohol, sondern mit Methylformiat unter Bildung von Methylmethacrylat und Formamid, umgesetzt wird.

Blausäure (HCN), welche für den ACH-Sulfo-Prozess und für die technische Herstellung verschiedener andere Produkte eingesetzt wird, kann technisch über verschiedene Wege gewonnen werden. Ausgehend von C1-Quellen sind die Umsetzung von Methan und Ammoniak in Gegenwart von Sauerstoff (Andrussow-Verfahren) oder in Abwesenheit von Sauerstoff (BMA-Verfahren) sowie die Ammoxidation von Methanol bekannt. Ausgehend von C3-Quellen kann Blausäure als Nebenprodukt bei der Ammoxidation von Propen zu Acrylnitril erhalten werden. Zudem kann Blausäure großtechnisch durch Dehydratisierung von Formamid hergestellt werden (BASF-Verfahren).

Im Andrussow-Verfahren wird Blausäure durch Ammoxidation von Methan mit Sauerstoff und Ammoniak, typischerweise an einem Platin-Katalysator, gemäß der Reaktionsgleichung CH₄ + NH₃+ 1,5 O₂ → HCN + 3 H₂O (Δ_{R}H = -481.06 kJ/mol) erhalten. Im BMA-Verfahren (Blausäure aus Methan und Ammoniak) der Degussa wird Blausäure aus Methan und Ammoniak durch Ammondehydrierung in Abwesenheit von Sauerstoff durch externe Energiezufuhr gemäß der Reaktionsgleichung CH₄ + NH₃ → HCN + 3 H₂ (Δ_{R}H = 251 kJ/mol) erhalten.

Im Andrussow- sowie im BMA-Verfahren wird typischerweise die Blausäure im erhaltenen Reaktionsgas nach Entfernung von Ammoniak mittels einer Schwefelsäure-Wäsche in einer nachgeschalteten Absorptionskolonne in Wasser absorbiert, um so eine Trennung von den ebenfalls im Reaktionsgas enthaltenen Inerten zu bewirken. Der Inert-Anteil, das sogenannte Armgas, enthält im wesentlichen Stickstoff, Wasserstoff und Kohlenmonoxid und kann beispielsweise energetisch verwertet werden. Die mit etwa 5 Gew.-% HCN beladene Aufnehmerphase, das sogenannte Absorberwasser, wird im Anschluss in eine Destillationskolonne geleitet, in der die Blausäure als Kopf-Produkt erhalten wird und der wässrige Sumpfstrom, meist nach einer Wärmeintegration, wieder als Absorberwasser zur Absorption der Blausäure eingesetzt wird.

Sowohl im Andrussow-Verfahren als auch im BMA-Verfahren reagiert Methan mit Ammoniak typischerweise bei Temperaturen von 1100 bis 1400 °C und liefert als Produkt HCN sowie Wasser H₂O (Andrussow) oder Wasserstoff H₂ (BMA). Methan wird typischerweise in Form von Erdgas oder eines methanreichen Gases aus einem Steamcracker bereitgestellt. Methanreiches Gas, das aus dem Spaltgas eines Steamcrackers isoliert wird, enthält normalerweise mehr als 90 Gew.-%, meist mehr als 95 Gew.-% Methan und weniger als 1 Gew.-% höhere Alkane, insbesondere Ethan sowie als typische weitere Komponenten Wasserstoff, Stickstoff und/oder andere Inerte. Der Methangehalt von Erdgas schwankt typischerweise zwischen 75 Vol.-% und 99 Vol.-%, während ein typischer Ethangehalt bzw. Propangehalt 0.1 - 15 Vol.-% bzw. 1 - 10 Vol.-% beträgt.

Bei der Herstellung von Blausäure führen die als Nebenkomponenten in der Methanquelle enthaltenen höheren Kohlenwasserstoffe unter den Bedingungen der Blausäuresynthese zur Bildung von Nitrilen. Ethan, Propan und Propen reagieren beispielsweise mit Ammoniak zu Acetonitril (ACN), Propionitril (PN) bzw. Acrylnitril (ACRN). Diese C2-C3 Nitrile stellen typische störende Nebenprodukte bei der HCN-Herstellung dar. Darüber hinaus können je nach Zusammensetzung der Methan-Quelle, in geringeren Mengen auch höhere Nitrile mit 4 bis 10 Kohlenstoffatomen als Nebenprodukte entstehen.

Typischerweise treten die folgenden Nitril-Nebenprodukte bei der HCN-Herstellung auf:

| | Siedepunkt [°C] |
|---|---|
| Acrylnitril (ACRN) | 77 |
| Acetonitril (ACN) | 82 |
| Propionitril (PN) | 97 |
| Butyronitril | 117 |
| Benzonitril | 191 |

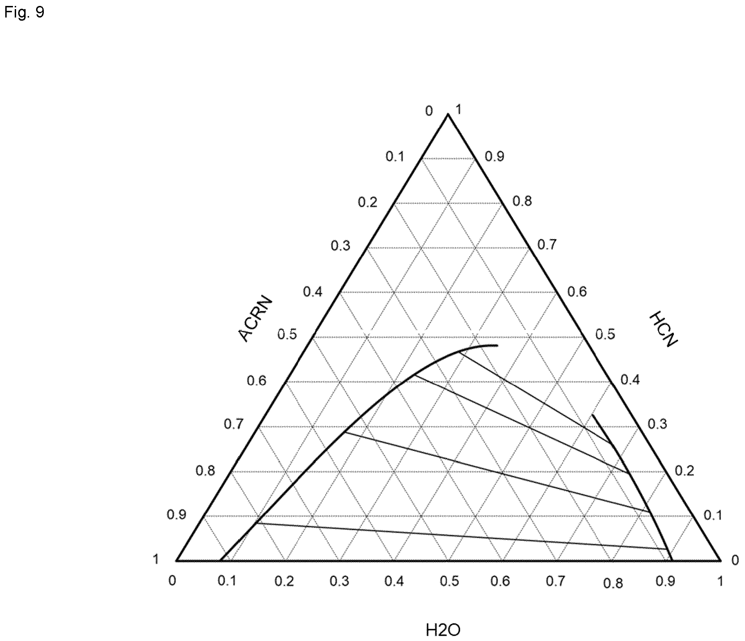

Diese Nitril-Nebenprodukte werden aufgrund ihrer Siedetemperaturen und Polarität zu einem großen Anteil in den Absorptionskolonnen zusammen mit der Blausäure in Wasser absorbiert und gelangen mit dem Absorberwasser in die Aufarbeitung. Die Nitrile besitzen teilweise Siedepunkte zwischen den beiden zu trennenden Hauptkomponenten Wasser (Siedepunkt 100 °C) und HCN (Siedepunkt 26 °C) und reichern sich daher bei der Rektifikation des Absorberwassers nach längerem Betrieb in den mittleren Stufen der entsprechenden Rektifikationskolonnen an. Dies ist insbesondere im Falle des Acrylnitrils problematisch, da nach Erreichen einer Acrylnitril-Grenzkonzentration von ca. 7 % ein ternäres Gemisch aus Blausäure, Wasser und Acrylnitril in eine wasserreiche, Acrylnitrilabgereicherte und eine blausäurereiche, Acrylnitril-angereicherte Phase zerfällt (siehe Figur 9). In der Acrylnitril-angereicherten Phase kann es zu einer Polymerisation des Acrylnitrils kommen, die störende Ablagerungen in der Destillationskolonne verursachen kann, was zu Stillständen und Ausfällen der Verfahrensanlage führen kann.

Während Acetonitril (ACN) und Wasser in der flüssigen Phase in jedem Verhältnis homogen mischbar sind, bilden Acrylnitril (ACRN) und Wasser über einen breiten Bereich von etwa 5-95 mol-% Acrylnitril ein zweiphasiges Gemisch. Betrachtet man die 3-Komponenten-Mischung aus ACRN, HCN und Wasser (Figur 9), tritt bei mehr als 10 Gew.-% ACRN und bei Wassergehalten über 50 Gew.-% 2-Phasigkeit auf. Zerfällt die flüssige Phase in der Destillationskolonne in zwei Phasen, kommt es verstärkt zur Polymerisation der Nitrile, insbesondere ACRN, da Stabilisatoren, die üblicherweise zur Verhinderung der Polymerisation zugegeben werden, in der wässrigen Phase verbleiben und somit die Polymerisation in der überwiegend organischen Phase nicht mehr wirksam verhindern können.

Darüber hinaus stellt auch die Polymerisation von Blausäure ein Problem dar, das zu störenden Ablagerungen in den Trennanlagen führen kann. Polymere Blausäure, auch Azulminsäure genannt, wird beispielsweise durch anionische Polymerisation gebildet. Dieser Mechanismus kann grundsätzlich auch in der Dampfphase in Trennapparaten starten, wobei sich die entstehende oligomere Blausäure bevorzugt an Oberflächen, festen Verunreinigungen oder bereits bestehenden Polymerisationskeimen, wie beispielweise Nitril-Polymer-Ablagerungen, im Trennapparat abscheidet. Die Polymerisation von Blausäure wird meist begünstigt durch Lichteinwirkung, lange Verweilzeiten, hohe Temperaturen, die Anwesenheit von Wasser und das Vorliegen eines erhöhten pH-Werts. Im alkalischen Milieu muss oft mit einer spontanen Polymerisation gerechnet werden. Polymere Blausäure tritt als braun-schwarzer, amorpher Feststoff in Erscheinung, der sich nur mit aggressiven Medien, wie beispielsweise konzentrierter Schwefelsäure oder Salpetersäure, auflösen lässt und mechanisch nur schwer entfernt werden kann. Zudem kann bei der Polymerisation der Nitril-Nebenprodukte, wie ACRN, Blausäure ebenfalls polymerisieren, wodurch sich die Menge der Ablagerungen weiter erhöht.

Die Blausäure-Polymerisation ist zudem von einer erheblichen Wärmeentwicklung begleitet, was zur Beschleunigung des Polymerisationsvorgangs führt und die Gefahr des Durchgehens der Reaktion besteht. So ist in E. H. Gause, P. D. Montgomery, J. Chem. Eng. Data 1960, 5 (3), 351-354, eine Polymerisationswärme von 42,6 kJ/mol angegeben.

Die Dokumente WO 2014/099568 A1 und US 2,656,251 A beschreiben Verfahren zur Herstellung von HCN nach Andrussow umfassend die Umsetzung von Methan, Ammoniak und Sauerstoff zu HCN in der Gasphase, Entfernen von nicht umgesetztem Ammoniak in einer sauren Absorption, Absorption von HCN in einem wässrigen Absorptionsmittel und die Abtrennung des HCN.

Die Bildung von Nitril-Polymeren und/oder Blausäure-Polymeren im Aufarbeitungsteil einer Anlage zur Herstellung von Blausäure beeinträchtigt in der Regel durch eine veränderte Strömungsdynamik in den Trennapparaten die Effizienz und somit die Kapazität des Aufarbeitungsteils. Damit wird die Effizienz des gesamten Blausäure-Herstellungsverfahrens beeinträchtigt. Die genannten Polymer-Ablagerungen müssen mit hohem mechanischem Aufwand entfernt werden oder machen oftmals sogar einen Austausch von kontaminierten Packungen oder Füllkörpern in Destillationskolonnen notwendig. Zudem verursachen die notwendige Öffnung und Begehung der Apparate aufgrund der hohen Toxizität von Blausäure einen hohen Spül- und Reinigungsaufwand. Insgesamt sind daher Aufwand, Dauer und Kosten der Unterbrechung für die Reinigung sehr hoch.

Im Stand der Technik werden Verfahren beschrieben, bei denen die Bildung der organischen Nitril-Nebenprodukte gezielt dahingehend genutzt wird, dass eine parallele Produktion von Blausäure und Nitrilen, insbesondere Acrylnitril (ACRN), mit gekoppelter Aufarbeitung betrieben werden können. So beschreibt das Dokument WO 2017/015521 A1 ein Verfahren, bei dem Blausäure in einem Andrussow-Prozess aus einer Erdgasquelle erzeugt wird, die einen Gehalt höherer Kohlenwasserstoffe von 0.05 bis 20 Gew.-% aufweist. Die entsprechend mit Nitrilen verunreinigte Blausäure wird mit einem ACRN-Produktstrom aus einer Propylen-Ammoxidationsanlage vereinigt und gemeinsam zunächst einer Ammoniak-Absorption Wäsche mit verdünnter Mineralsäure bei pH < 7 unterworfen. Die im Ammoniak-abgereicherten Prozessgas enthaltenen Nitrile sowie die Blausäure werden anschließend in einer weiteren Absorptionsvorrichtung in die Wasserphase überführt und zu Acrylnitril und Blausäure aufgearbeitet. Die Druckschrift beschreibt jedoch keine Details der Aufarbeitung und Rückführung von Stoffströmen oder zur erzielbaren Reinheit der Blausäure. Eine Verschaltung gemäß WO 2017/015521 A1 ist zudem nur praktikabel, wenn sowohl Blausäure als auch ACRN, welches als Monomer-Rohstoff für verschiedene (Co-) Polymere dient, als Produkte gewonnen werden sollen.

Das Dokument WO 2004/092068 beschreibt die Destillation eines wässrigen Blausäure-Rohprodukts enthaltend 50 bis 99,9 Gew.-% HCN, 0,1 bis 40 Gew.-% Wasser, 0 bis 15 Gew.-% Kohlenstoffoxide und optional 0.01 bis 1 Gew.-% eines schwerflüchtigen Stabilisators, z.B. Phosphorsäure in Abwesenheit eines leichtflüchtigen Stabilisators. Die Destillation wird bevorzugt in einer Glockenbodenkolonne bei definiertem Druck und definierten Sumpf- und Kopftemperaturen durchgeführt. Als Kopfprodukt wird eine gereinigte, wasserfrei Blausäure erhalten. Im Sumpf wird ein Strom, enthaltend Wasser und optional den schwerflüchtigen Stabilisator, abgezogen. Dieses Verfahren wird vorzugsweise zur Entwässerung eines wasserhaltigen Blausäure-Rohproduktes, welches durch thermische Spaltung von Formamid erhalten wird, eingesetzt. Das wasserfreie Blausäure-Produkt soll über mehrere Tage bei 5 bis 25 °C in Abwesenheit eines Stabilisators gelagert werden können. Das gereinigte, weitgehend wasserfrei Blausäure-Produkt wird bevorzugt in einem Verfahren zur Herstellung von Nitrilen, z.B. Adipinsäuredinitril, durch Hydrocyanierung von Olefinen oder Dienen, z.B. von Butadien, verwendet. Die Anreicherung und/oder die gezielte Ausschleusung von Nitril-Nebenprodukten wird hier nicht beschrieben.

Das Dokument WO 2017/011428 A1 beschreibt ein Verfahren zur Reinigung von Blausäure, wobei die Anreicherung von Nitril-Nebenprodukten in der Destillationskolonne zur Trennung von Blausäure und Wasser und der Verlust an HCN durch eine Nitril-Ausschleusung vermindert werden sollen. Das hier beschriebene Verfahren umfasst das Abführen eines Seitenstroms aus der Destillationskolonne, wobei dieser Seitenstrom in einer separaten Stripp-Kolonne zur Trennung von Blausäure von Nitrilen und Wasser behandelt wird. Die gereinigte Blausäure aus dem Kopf der Stripp-Seitenkolonne wird zurück in die Hauptdestillationskolonne gefahren, der wässrige Strom enthaltend Nitrile wird aus dem Verfahren ausgeschleust. Nachteilig ist hier, dass ein zusätzlicher apparativer Aufwand notwendig ist und dass eine große Menge an wässrigem, Nitril-haltigem Abfall entsorgt werden muss.

Das Dokument EP 3 604 222 A1 beschreibt ein Verfahren zur Reinigung von Blausäure und ein Verfahren zur Herstellung von 2-Hydroxy-4-(methylthio)butyronitril durch Umsetzung von Blausäure und 3-Mercaptopropionaldehyd. In dem in EP 3 604 222 A1 beschriebenen Reinigungsverfahren wird das Blausäure-Rohprodukt, welches nach dem Andrussow-Verfahren oder dem BMA-Verfahren erhalten werden kann, in zwei Ströme aufgeteilt und an verschiedenen Stellen der Destillationskolonne zugeführt. Hierbei wird der erste Strom mit einer Temperatur T1 zwischen Kopf und Sumpf der Destillationskolonne zugeführt und der zweite Strom mit einer Temperatur T2 am Kopf der Destillationskolonne zugeführt, wobei die Temperatur T2 geringer ist als die Temperatur T1. Beispielsweise kann der erste Zulaufstrom in einem Wärmetauscher unter Verwendung des Sumpfablaufs der Destillationskolonne vorgeheizt werden. Durch diese Stoffstrom- und Temperaturführung soll die Anreicherung von Nitril-Nebenprodukten in der Destillationskolonne vermindert werden. Das hier beschriebene Verfahren verlangt ebenfalls einen hohen zusätzlichen apparativen Aufwand, um die Anreicherung von Nitril-Nebenprodukten zu vermindern.

Keines der Verfahren im Stand der Technik beschreibt eine Möglichkeit zur Kontrolle und gezielten Ausschleusung von störenden Nitril-Nebenprodukten, insbesondere von ACN, ACRN und PN. Somit besteht weiterhin ein hoher Bedarf an einem großtechnischen Verfahren zur Herstellung von Blausäure in hoher Ausbeute, das einfach und kostengünstig durchzuführen ist, und bei dem eine Anreicherung von Nitrilen und die daraus resultierenden Nachteile hinsichtlich Produktqualität und Betriebssicherheit durch eine optimierte Verfahrensführung vermindert werden.

### Aufgabe der Erfindung

Aufgabe der vorliegenden Erfindung war es, die oben genannten Nachteile zu überwinden und ein wirtschaftliches Verfahren zu finden für die Methan-basierte Herstellung von Blausäure mit hoher Reinheit sowie mit einem niedrigen Gehalt an höheren Nitrilen, die eine ähnliche oder erhöhte Flüchtigkeit aufweisen wie das Zielprodukt und die durch konventionelle Trennsequenzen nicht oder nur mit erheblichem Aufwand vom Zielprodukt abgetrennt werden können. Aufgabe war es insbesondere, durch eine geeignete Verfahrensführung die Bereitstellung von Blausäure mit einem Gesamtgehalt an Nitrilen (wie z.B. Acetonitril, Propionitril und Acrylnitril) geringer als 5.000 ppm und einem Wassergehalt geringer als 10.000 ppm zu gewährleisten. Insbesondere war es wichtig, dass der Gehalt an Nitrilen so weit reduziert ist, dass in den Nachfolgeprozessen, in denen die Blausäure als Einsatzstoff verwendet wird, negative Auswirkungen vermieden werden.

Eine Aufgabe dieser Erfindung ist es, die Aufarbeitung des Blausäure-Produkts und die Stoffstromführungen im Gesamtverfahren zu optimieren, um eine Anreicherung von Nitrilen und deren Polymerisation zu vermeiden. Zudem soll eine hohe Trennschärfe von Wasser und HCN und damit ein HCN-Reinprodukt mit geringem Wasseranteil erreicht werden. Hierbei soll bevorzugt auf eine aufwändige Verfahrensführungen und zusätzliche Apparate, wie etwa eine Seitenstrombehandlung, verzichtet werden.

Außerdem soll im Vergleich zu bekannten Verfahren eine vergleichbare oder erhöhte Ausbeute an Blausäure erhalten werden. Weitere, nicht explizit genannte Aufgaben, können sich aus der Beschreibung und den Beispielen der Erfindung ergeben.

### Lösung der Aufgabe

Es wurde überraschend gefunden, dass die oben genannten Aufgaben durch das erfindungsgemäße Verfahren gelöst werden. Die Erfindung stellt ein verbessertes Verfahren zur Herstellung und Aufarbeitung von Blausäure (HCN) bereit, umfassend die Aufarbeitung und Entwässerung des HCN-Rohprodukts durch Destillation, wobei eine optimierte Rückführung und Ausschleusung von Nitril-angereichten Stoffströmen verwirklicht ist. Insbesondere wurde gefunden, dass die Menge an störenden organischen Nitril-Nebenprodukten im Herstellprozess und im Produkt reduziert werden kann. Insbesondere kann das Anreichern von Nitrilen in der Destillationskolonne vermindert werden, wodurch die Polymerisation der Nitrile und ggf. auch die der Blausäure sowie schädliche Ablagerungen vermindert werden können.

Insgesamt gelingt es mit Hilfe des erfindungsgemäßen Verfahrens, die Herstellung von Blausäure sicherer, weniger störungsanfällig und mit höheren Ausbeuten durchzuführen, wobei die Abtrennung des Blausäure-Produktes in der erforderlichen Qualität effektiv möglich ist.

### Beschreibung der Erfindung

Die vorliegende Erfindung betrifft ein Verfahren zur Herstellung von Blausäure (HCN), umfassend die Schritte
i. Umsetzung einer Methan-Quelle (z.B. 1), einer Ammoniak-Quelle (z.B. 2) und optional einer Sauerstoff-Quelle (z.B. 3) in einem Reaktor (z.B. A) in einem Reaktionsteil I unter Verwendung eines platinhaltigen Kontaktes, wobei ein HCN-haltiges Prozessgas (z.B. 4) erhalten wird, welches Nitril-Nebenprodukte, insbesondere ausgewählt aus C2-C10 Nitrilen, insbesondere ausgewählt aus Acetonitril, Acrylnitril und Propionitril, enthält;
ii. Absorption von nicht umgesetztem Ammoniak in einem Aufarbeitungsteil II, wobei das HCN-haltige Prozessgas (z.B. 4) in mindestens einer ersten Absorptionsvorrichtung (z.B. B), mit einem sauren, wässrigen Absorptionsmittel behandelt wird, wobei ein Ammoniak-abgereichertes Prozessgas (z.B. 5) und eine wässrige Ammoniumsulfat-haltige Lösung (z.B. 6) erhalten werden;
iii. Absorption von HCN im Aufarbeitungsteil II, wobei das Ammoniak-abgereicherte Prozessgas (z.B. 5) in mindestens einer zweiten Absorptionsvorrichtung (z.B. C) mit einem wässrigen Absorptionsmittel behandelt wird, wobei eine erste wässrige HCN-haltige Lösung (z.B. 8) und ein HCN-abgereichertes Armgas (z.B. 7) erhalten werden;
iv. Erwärmen der ersten wässrigen HCN-haltigen Lösung (z.B. 8) im Aufarbeitungsteil II in mindestens einer Wärmeaustauschapparatur (z.B. D), wobei eine zweite wässrige HCN-haltige Lösung (z.B. 10) erhalten wird;
v. Destillation der zweiten wässrigen HCN-haltigen Lösung (z.B. 10) im Aufarbeitungsteil II in mindestens einer Destillationsvorrichtung (z.B. E), wobei ein HCN-Reinstrom (z.B. 11) und ein Nitril-haltiger, wässriger Sumpfstrom (z.B. 12) erhalten werden;
wobei
a. die Methan-Quelle (1) mindestens 87,0 Vol.-% Methan; 0,2 bis 8,0 Vol.-% C2-C10 Alkane und optional bis zu 5,0 Vol.-% Inerte, beispielsweise ausgewählt aus Stickstoff, Wasserstoff und Kohlenoxiden, jeweils bezogen auf die gesamte Methan-Quelle (1), enthält;
b. die im HCN-haltigen Prozessgas enthaltenen Nitril-Nebenprodukte, insbesondere ausgewählt aus C2-C10 Nitrilen, insbesondere ausgewählt aus Acetonitril, Acrylnitril und Propionitril, durch die (bzw. alle der) folgenden Schritte aus dem Verfahren ausgeschleust werden
   indem die wässrige Ammoniumsulfat-haltige Lösung (z.B. 6), welche in Schritt ii) erhalten wird, und welche einen pH-Wert im Bereich von 2 bis 5 aufweist, vollständig oder teilweise aus dem Verfahren ausgeschleust wird;
   indem der Nitril-haltige, wässrige Sumpfstrom (z.B. 12), welcher in Schritt v) erhalten wird, zumindest teilweise aus dem Verfahren ausgeschleust wird (z.B. 13a) und/oder zumindest teilweise in die erste Absorptionsvorrichtung (z.B. B) in Schritt ii) zurückgeführt wird (z.B. 13c);
   indem der HCN-Reinstrom (z.B. 11), welcher in Schritt v) erhalten wird, 500 bis 5.000 ppm, bevorzugt 600 bis 4.500 ppm, besonders bevorzugt 800 bis 4.500 ppm, C2-C10-Nitrile, insbesondere ausgewählt aus Acetonitril, Acrylnitril und Propionitril, und 10 bis 10.000 ppm, bevorzugt 100 bis 7.000 ppm, besonders bevorzugt 200 bis 6.000 ppm, Wasser, jeweils bezogen auf den gesamten HCN-Reinstrom, enthält;
   und indem der Nitril-haltige, wässrige Sumpfstrom (z.B. 12), welcher in Schritt v) erhalten wird, 10 bis 1.800 ppm, bevorzugt 50 bis 1.600 ppm, besonders bevorzugt 100 bis 1.500 ppm, C2-C10-Nitrile, insbesondere ausgewählt aus Acetonitril, Acrylnitril und Propionitril, bezogen auf den gesamten wässrige Sumpfstrom, enthält.

Im Sinne der vorliegenden Erfindung meint der Ausdruck "ppm", ohne weitere Angaben, Gew.-ppm (z.B. mg/kg).

Der Ausdruck Strom, Phase oder Fraktion enthaltend ein Edukt, Produkt und / oder Nebenprodukt ist im Sinne der Erfindung so zu verstehen, dass die genannte(n) Verbindung(en) in dem jeweiligen Strom enthalten ist (sind); beispielsweise ist der überwiegende Anteil des Edukts, Produkts und / oder Nebenprodukts in dem entsprechenden Strom zu finden. Grundsätzlich können neben den genannten Verbindungen weitere Bestandteile enthalten sein. Oftmals dient die Nennung der Bestandteile der Verdeutlichung des jeweiligen Verfahrensschritts.

Bevorzugt handelt es sich bei dem erfindungsgemäßen Verfahren um ein kontinuierliches oder halbkontinuierliches Verfahren. Besonders bevorzugt ist das erfindungsgemäße Verfahren ein kontinuierliches Verfahren.

Typischerweise beziehen sich die Mengenangaben und Mengenbereiche betreffend C2-C10-Nitrile auf die Summe aller enthaltenen C2-C10-Nitrile. Bevorzugt handelt es sich hierbei um C2-C10-Nitrile, welche in nachweisbarer Menge in den Stoffströmen vorhanden sind. Bevorzugt handelt es sich um Acetonitril, Acrylnitril und/oder Propionitril, wobei sich die Mengenangaben und Mengenbereiche betreffend C2-C10-Nitrile auf die Summe aus Acetonitril, Acrylnitril und Propionitril beziehen.

Bevorzugt werden der erfindungsgemäße Gehalt an C2-C10-Nitrilen und der Wassergehalt im HCN-Reinstrom, welcher in Schritt v) erhalten wird, sowie der erfindungsgemäße Gehalt an C2-C10-Nitrilen im Nitril-haltige, wässrige Sumpfstrom, welcher in Schritt v) erhalten wird, durch Einstellen von einem oder mehreren Parametern der Destillation in Schritt v. erreicht, insbesondere von einem oder mehreren Parameter ausgewählt aus Temperaturverlauf, Anzahl der theoretischen Stufen, Lage des Zulaufs, Rücklaufverhältnis (d.h. das Verhältnis aus Rücklauf-Strom und ausgeschleustem Destillat-Strom), Kondensationsleistung am Kopf der Destillationsvorrichtung, Heizleistung der Destillationsvorrichtung und Druck in der Destillationsvorrichtung. Beispielweise kann durch Erhöhung des Rücklaufverhältnisses in der Destillationsvorrichtung in Schritt v) in der Regel die Konzentrationen an Nitrilen am Kopf der Destillationsvorrichtung reduziert werden. Beispielweise kann durch Erhöhung der Heizleistung in der Destillationsvorrichtung in Schritt v) in der Regel die Konzentrationen an Nitrilen am Sumpf der Destillationsvorrichtung reduziert werden.

Der Gehalt an C2-C10-Nitrilen sowie der Gehalt an Wasser in den verschiedenen Stoffströmen kann in bekannter Weise anhand geeigneter Analysemethoden, beispielsweise mittels Hochleistungsflüssigkeitschromatographie (High Performance Liquid Chromatography HPLC) oder Gas-Chromatographie (GC), insbesondere mittels Gas-Chromatographie mit gekoppelter Massenspektrometrie (GC-MS), ermittelt werden.

### HCN-Synthese / Schritt i)

Das erfindungsgemäße Verfahren umfasst als Schritt i) in einem Reaktionsteil I die Umsetzung einer Methan-Quelle, beispielsweise Erdgas, einer Ammoniak-Quelle und optional einer Sauerstoff-Quelle, beispielsweise Luft, in einem Reaktor (z.B. A) unter Verwendung eines platinhaltigen Kontaktes , wobei ein HCN-haltiges Prozessgas (z.B. 4) erhalten wird, welches bevorzugt 5 bis 50 Vol.-% HCN und Nitril-Nebenprodukte, insbesondere ausgewählt aus C2-C10 Nitrilen, insbesondere Acetonitril, Propionitril und Acrylnitril, enthält.

Typischerweise handelt es sich bei der Methan-Quelle (z.B. 1) um ein gasförmiges Gemisch enthaltend Methan und meist ein oder mehrere C2-C10 Alkane, insbesondere Ethan und Propan. Beispielsweise kann es sich bei der Methan-Quelle um Erdgas oder ein Methanreiches Gas erhalten aus dem Spaltgas eines Steam-Crackers handeln.

Erfindungsgemäß enthält die Methan-Quelle, die in Schritt i) eingesetzt wird, mindestens 87,0 Vol.-% Methan, 0,2 bis 8,0 Vol.-% C2-C10 Alkane und optional bis zu 5,0 Vol.-% Inerte, beispielsweise ausgewählt aus Stickstoff, Wasserstoff und Kohlenoxiden, jeweils bezogen auf die gesamte Methan-Quelle. Bevorzugt enthält die Methan-Quelle 87,0 bis 99,8 Vol.-% Methan; 0,2 bis 5,0 Vol.-% Ethan, 0,0 bis 3,0 Vol.-% Propan und 0,0 bis 5,0 Vol.% Inerte, beispielsweise ausgewählt aus Stickstoff, Wasserstoff und Kohlenoxiden, jeweils bezogen auf die gesamte Methan-Quelle. Weiterhin bevorzugt kann die Methan-Quelle 90,0 bis 99,8 Vol.-% Methan, 0,19 bis 5 Vol.-% Ethan, 0,01 bis 3 Vol.-%, Propan und optional bis zu 5,0 Vol.% Inerte, beispielsweise ausgewählt aus Stickstoff, Wasserstoff und Kohlenoxiden, jeweils bezogen auf die gesamte Methan-Quelle, enthalten.

Der im Reaktionsteil I in Reaktor A erhaltene Produktstrom (HCN-haltiges Prozessgas) (z.B. 4) enthält bevorzugt 6 bis 30 Vol.-%, besonders bevorzugt 8 bis 25 Vol.-%, HCN und typischerweise 1 bis 10.000 ppm, bevorzugt 500 bis 5.000 ppm Nitril-Nebenprodukte, bezogen auf das HCN-haltiges Prozessgas. Im Falle des Andrussow-Verfahrens enthält das HCN-haltiges Prozessgas (z.B. 4) bevorzugt 6 bis 20 Vol.-%, besonders bevorzugt 6 bis 15 Vol.-% HCN und typischerweise 1 bis 10.000 ppm, bevorzugt 500 bis 5.000 ppm Nitril-Nebenprodukte, bezogen auf das HCN-haltiges Prozessgas. Im Falle des BMA-Verfahrens enthält das HCN-haltiges Prozessgas (z.B. 4) bevorzugt 10 bis 30 Vol.-%, besonders bevorzugt 15 bis 25 Vol.-% HCN und typischerweise 1 bis 10.000 ppm, bevorzugt 500 bis 5.000 ppm Nitril-Nebenprodukte, bezogen auf das HCN-haltiges Prozessgas.

Typischerweise werden bei der HCN-Synthese in Schritt i) Nitrile, insbesondere aliphatische und aromatische Nitrile umfassend 2-10 Kohlenstoffatome, beispielsweise Acrylnitril, Acetonitril, Propionitril, Butyronitril und Benzonitril, insbesondere Acetonitril, Propionitril und/oder Acrylnitril, gebildet.

Bevorzugt enthält das HCN-haltige Prozessgas (z.B. 4), welches in Schritt i) erhalten wird, 1 bis 10.000 ppm, bevorzugt 10 bis 8.000 ppm, bevorzugt 100 bis 6.000 ppm, besonders bevorzugt 500 bis 5.000 ppm Nitril-Nebenprodukte, bevorzugt ausgewählt aus C2-C10-Nitrilen, insbesondere ausgewählt aus Acetonitril, Acrylnitril und Propionitril. Diese organischen Nitril-Nebenprodukte weisen oftmals Siedepunkte im Bereich von 26 °C und 100 °C und damit zwischen jenen der zu trennenden Stoffen, nämlich Blausäure und Wasser, auf. Folglich können sich diese Nitrile in einer zur Aufarbeitung der Blausäure eingesetzten Destillationskolonne anreichern und zu Polymer-Ablagerungen führen, wie vorne beschrieben.

Die Durchführung der Blausäure-Herstellung nach dem Andrussow-Verfahren oder dem BMA-Verfahren sind dem Fachmann bekannt und in zahlreichen Standardwerken beschrieben, z.B. Asendorf, E. & Klempt, W., Cyanverbindungen, Ullmanns Encyklopädie der technischen Chemie, Urban & Schwarzenberg, 1954, 626-669; Weigert, W.; Düsing, G.; Knorre, H.; Kriebitzsch, N. & Pfleger, H., Cyan-Verbindungen, Ullmanns Encyklopädie der technischen Chemie, Verlag Chemie, 1975, 655-673; Düsing, G. & Pfleger, H., Blausäure und Cyanide, Winnacker-Küchler; Chemische Technologie, Band 2: Anorganische Technologie I, Carl Hanser Verlag, 1982, 189-203; Klenk, H.; Griffiths, A.; Huthmacher, K.; Itzel, H.; Knorre, H.; Voigt, C., Cyano compounds, inorganic, Ullmann's Encyclopedia of Industrial Chemistry, VCH Verlagsgeselschaft, 1987, 159; Sauer, J.; Bewersdorf, M.; Köstner, M.; Rinner, M. & Wolf, D., Hydrocyanic Acid (HCN) Production, Handbook of heterogeneous catalysis, Wiley, 2008, 2592-2609; Gail, E.; Gos, S.; Kulzer, R.; Lorösch, J.; Rubo, A.; Sauer, M.; Kellens, R.; Reddy, J.; Steier, N. & Hasenpusch, W., Cyano Compounds, Inorganic, Ullmann's Encyclopedia of Industrial Chemistry, Wiley-VCH, 2011, 10, 673-710; Maxwell, G.; Allison, J. & Dixon, R., Cyanides, Kirk-Othmer Encyclopedia of Chemical Technology, Wiley, 2021, 1-42.

### Erste Absorption / Schritt ii)

Als Schritt ii) umfasst das erfindungsgemäße Verfahren die Absorption von nicht umgesetztem Ammoniak in einem Aufarbeitungsteil II, wobei das HCN-haltige Prozessgas (z.B. 4) in mindestens einer ersten Absorptionsvorrichtung (z.B. B) mit einem sauren, wässrigen Absorptionsmittel behandelt wird, wobei ein Ammoniak-abgereichertes Prozessgas (z.B. 5) und eine wässrige Ammoniumsulfat(AMSUL)-haltige Lösung (z.B. 6) erhalten werden.

In einer bevorzugten Ausführungsform wird die wässrige Ammoniumsulfat-haltige Lösung (z.B. 6), welche in Schritt ii) erhalten wird, vollständig aus dem Verfahren ausgeschleust und kann optional einer Aufreinigung wie unten beschrieben unterzogen werden.

Die im HCN-haltigen Prozessgas enthaltenen Nitril-Nebenprodukte, insbesondere Acetonitril, Propionitril und Acrylnitril, werden zumindest teilweise aus dem Verfahren ausgeschleust, indem die wässrige Ammoniumsulfat-haltige Lösung (z.B. 6), welche in Schritt ii) erhalten wird und welche einen pH-Wert im Bereich von 2 bis 5; bevorzugt 2,5 bis 4,5; besonders bevorzugt 3 bis 4, aufweist, vollständig oder teilweise aus dem Verfahren ausgeschleust wird.

In einer bevorzugten Ausführungsform enthält die wässrige Ammoniumsulfat-haltige Lösung (z.B. 6), welche in Schritt ii) erhalten wird, 10 bis 1.000 ppm, bevorzugt 10 bis 700 ppm, bezogen auf die gesamte wässrige Ammoniumsulfat-haltige Lösung, C2-C10-Nitrile, insbesondere ausgewählt aus Acetonitril, Acrylnitril und Propionitril, wobei die wässrige Ammoniumsulfat-haltige Lösung, optional nach einer weiteren Aufarbeitung/Reinigung, vollständig aus dem Verfahren ausgeschleust wird.

Bevorzugt wird als saures, wässriges Absorptionsmittel in Schritt ii) eine wässrige Mischung mindestens einer Brönsted-Säure, insbesondere Schwefelsäure, eingesetzt. Als saures, wässriges Absorptionsmittel können Frischsäure, insbesondere eine Schwefelsäure-Quelle (z.B. 16) und/oder ein zurückgeführter wässriger Stoffstrom (z.B. 15), dem typischerweise Frischsäure, insbesondere eine Schwefelsäure-Quelle (z.B. 14) zugegeben wird, verwendet werden. Typischerweise weist das saure, wässrige Absorptionsmittel in Schritt ii) einen pH-Wert im Bereich von 1 bis 4, bevorzugt 2 bis 3 auf. Bevorzugt erfolgt die Zugabe von Frischsäure, insbesondere Schwefelsäure, direkt in die erste Absorptionsvorrichtung (B) (z.B. über 16) oder in den Nitril-haltigen, wässrigen Sumpfstrom, welcher in Schritt v) erhalten wird und teilweise in die erste Absorptionsvorrichtung (B) zurückgeführt wird (z.B. über 14).

Bevorzugt wird Menge und pH-Wert des sauren, wässrigen Absorptionsmittels so gewählt, dass die wässrige AMSUL-haltige Lösung (z.B. 6), welche aus der ersten Absorptionsvorrichtung (z.B. B) abgeführt wird, einen pH-Wert im Bereich von 2 bis 5; bevorzugt 2,5 bis 4,5; besonders bevorzugt 3 bis 4, aufweist.

### Zweite Absorption / Schritt iii)

Das erfindungsgemäße Verfahren umfasst in Schritt iii) die Absorption von HCN, wobei das Ammoniak-abgereicherte Prozessgas (z.B. 5) in mindestens einer zweiten Absorptionsvorrichtung (z.B. C) mit einem wässrigen Absorptionsmittel behandelt wird, wobei eine erste wässrige HCN-haltige Lösung (z.B. 8) und ein HCN-abgereichertes Armgas (z.B. 7) erhalten werden.

In einer bevorzugten Ausführungsform wird in Schritt iii) das Ammoniak-abgereicherte Prozessgas (z.B. 5) bei 0° bis 20°C und bei einem Druck von 1 bara bis 10 bara mit dem wässrigen Absorptionsmittel behandelt, wobei die erste wässrige HCN-haltige Lösung (z.B. 8) erhalten wird. Typischerweise enthält diese erste wässrige HCN-haltige Lösung (z.B. 8) 1 bis 50 Gew.-% HCN; 50 bis 98,9999 Gew.-% Wasser, und 1 bis 5.000 ppm, bevorzugt 1 bis 2.000 ppm an Nitril-Nebenprodukten, insbesondere ausgewählt aus C2-C10 Nitrilen, jeweils bezogen auf die gesamte erste wässrige HCN-haltige Lösung.

Das in Schritt iii) erhaltene Armgas (z.B. 7) enthält bevorzugt, je nach Reaktionsführung in Reaktor A, 10 bis 10.000 ppm an C2-C10 Nitril-Nebenprodukten, insbesondere ausgewählt aus Acetonitril, Acrylnitril und Propionitril, jeweils bezogen auf das gesamte Armgas. Darüber hinaus enthält das Armgas typischerweise je nach Reaktionsführung in Reaktor A Wasserstoff und/oder Kohlenoxide.

Bevorzugt wird als wässriges Absorptionsmittel in der zweiten Absorptionsvorrichtung (z.B. C) ein Teil des Nitril-haltigen, wässrigen Sumpfstroms (z.B. 12), welcher in Schritt v) erhalten wird, und welcher optional über die Wärmeaustauschapparatur (z.B. D) geführt wird, verwendet. Typischerweise weist das wässriges Absorptionsmittel in der zweiten Absorptionsvorrichtung (z.B. 9) einen pH-Wert im Bereich von 2.5 bis 7, bevorzugt 3 bis 5, auf.

### Erwärmen in Wärmeaustauschapparatur / Schritt iv)

Das erfindungsgemäße Verfahren umfasst in Schritt iv) das Erwärmen der ersten wässrigen HCN-haltigen Lösung (z.B. 8) in mindestens einer Wärmeaustauschapparatur (z.B. D), wobei eine zweite wässrige HCN-haltige Lösung (z.B. 10) erhalten wird. Hierbei können typische und dem Fachmann bekannte Bauformen für Wärmeaustauschapparatur, wie beispielsweise Rohr-Wärmeaustauscher, Rohrbündel-Wärmeaustauscher, Platten-Wärmeaustauscher, und Mantelrohr-Wärmeaustauscher, in Gegenstrom-, Gleichstrom- und/oder Kreuzstrom-Führung verwendet werden.

In einer bevorzugten Ausführungsform wird der Nitril-haltige, wässrige Sumpfstrom (z.B. 12), welcher in Schritt v) erhalten wird, zumindest teilweise in die zweite Absorptionsvorrichtung (z.B. C) in Schritt iii) zurückgeführt. Dies kann beispielsweise direkt erfolgen oder über den Wärmetauscher D (z.B. über 13b und 9). Insbesondere bevorzugt wird dieser zurückgeführte Nitril-haltige, wässrige Sumpfstrom (z.B. 13b) über die mindestens eine Wärmeaustauschapparatur (z.B. D) geführt, um die erste wässrige HCN-haltige Lösung (z.B. 8), welche in Schritt iii) erhalten wird, zu erwärmen. Typischerweise weist dieser zurückgeführte Nitril-haltige, wässrige Sumpfstrom (z.B. 13b) eine Temperatur im Bereich von 80 bis 100 °C auf.

Typischerweise weist die zweite wässrige HCN-haltige Lösung (z.B. 10), welche nach dem Wärmetauscher-Schritt iv) erhalten wird und welche als Roh-Blausäure-Zulauf in die mindestens eine Destillationsvorrichtung (z.B. E) geführt wird, eine Temperatur im Bereich von 60 bis 95 °C, bevorzugt 70 bis 90 °C, auf.

Bevorzugt enthält die zweite wässrige HCN-haltige Lösung (z.B. 10), welche in Schritt iv) erhalten wird und welche als Roh-Blausäure in die Destillationsvorrichtung des Schritts v) geführt wird, 1 bis 50 Gew.-% HCN, 50 bis 98.9999 Gew.-% Wasser, und 1 bis 5.000 ppm an Nitril-Nebenprodukten, insbesondere ausgewählt aus C2-C10 Nitrile, jeweils bezogen auf die gesamte zweite wässrige HCN-haltige Lösung.

### Aufreinigung HCN in Destillation / Schritt v)

Das erfindungsgemäße Verfahren umfasst in Schritt v) die Destillation der zweiten wässrigen HCN-haltigen Lösung (z.B. 10) im Aufarbeitungsteil II in mindestens einer Destillationsvorrichtung (z.B. E), wobei ein HCN-Reinstrom (11) und ein Nitril-haltiger, wässriger Sumpfstrom (z.B. 12) erhalten werden.

Erfindungsgemäß ist die vorteilhafte Ausschleusung der im HCN-haltigen Prozessgas (z.B. 4) enthaltenen C2-C10 Nitril-Nebenprodukte, insbesondere ausgewählt aus Acetonitril, Acrylnitril und Propionitril, aus dem Verfahren unter anderem dadurch gewährleistet, dass der Destillationsschritt v) so geführt wird, dass der HCN-Reinstrom (z.B. 11), welcher in Schritt v) erhalten wird, 500 bis 5.000 ppm, bevorzugt 600 bis 4.500 ppm, besonders bevorzugt 800 bis 4.500 ppm, C2-C10-Nitrile, insbesondere ausgewählt aus Acetonitril, Acrylnitril und Propionitril, und 10 bis 10.000 ppm, bevorzugt 100 bis 7.000 ppm, besonders bevorzugt 200 bis 6.000 ppm, Wasser, jeweils bezogen auf den gesamten HCN-Reinstrom, enthält; und der Nitril-haltige, wässrige Sumpfstrom (z.B. (12)), welcher in Schritt v) erhalten wird, 10 bis 1.800 ppm, bevorzugt 50 bis 1.500 ppm, besonders bevorzugt 100 bis 1.500 ppm C2-C10-Nitrile, insbesondere ausgewählt aus Acetonitril, Acrylnitril und Propionitril, bezogen auf den gesamten wässrige Sumpfstrom, enthält.

Insbesondere kann die Verteilung der C2-C10-Nitrile und der Wassergehalt im Kopfstrom (HCN-Reinstrom) in der Destillationsvorrichtung (z.B. E) durch geeignete Einstellung von einem oder mehreren Parametern, wie sie dem Fachmann bekannt sind, erzielt werden, beispielsweise durch gezielte Einstellung eines oder mehrerer Parameter ausgewählt aus Temperaturverlauf, Anzahl der theoretischen Stufen, Zulauf, Rücklaufverhältnis, Kondensations- und Heizleistung und Druck.

Bevorzugt handelt es sich bei der Destillationsvorrichtung (z.B. E) um eine Destillationskolonne, insbesondere eine Destillationskolonne mit Packungen oder Böden oder jeweils zu Teilen mit Packung und/oder Böden. Bevorzugt handelt es sich bei der Destillationsvorrichtung (z.B. E) um eine Destillationskolonne umfassend 15 bis 50, bevorzugt 20 bis 40, insbesondere bevorzugt 25 bis 40, theoretische Böden.

In einer bevorzugten Ausführungsform wird in Schritt v) die Destillation der zweiten wässrigen HCN-haltigen Lösung (z.B. 10) bei einem Druck von 500 mbar bis 3000 mbar absolut, bevorzugt 500 mbar bis 1500 mbar absolut, in mindestens einer Destillationskolonne durchgeführt, wobei die gemittelte Temperatur im mittleren Bereich der Destillationskolonne bevorzugt im Bereich von 45 bis 97 °C, bevorzugt 60 bis 96 °C, liegt. Typischerweise kann eine geeignete gemittelte Temperatur im mittleren Bereich der Destillationsvorrichtung in Abhängigkeit der Ausgestaltung der Destillationsvorrichtung (E), z.B. Anzahl der theoretischen Böden der Destillationskolonne, gewählt werden. Hierbei bezieht sich der mittlere Teil der Destillationskolonne typischerweise auf das mittlere Drittel der Höhe bzw. Anzahl der theoretischen Stufen der Destillationskolonne. Ausgehend von einer Anzahl an theoretischen Stufen der Destillationskolonne nₘₐₓ bezieht sich diese gemittelte Temperatur auf die Temperatur gemittelt über die Böden n₁ bis n₂, mit n₁=0,33 * nₘₐₓ und n₂= 0,66 * nₘₐₓ.

In einer bevorzugten Ausführungsform umfasst die Destillationsvorrichtung (E), typischerweise die Destillationskolonne (E), 25 bis 45, bevorzugt 30 bis 45, theoretische Böden, und die gemittelte Temperatur im mittleren Bereich der Destillationskolonne liegt im Bereich von 45 bis 90 °C, bevorzugt 60 bis 85 °C.

Weiterhin bevorzugt erfolgt die Destillation in Schritt v) in mindestens einer Destillationskolonne, wobei die Sumpftemperatur der Destillationskolonne (z.B. E) im Bereich von 80 bis 110°C, bevorzugt 90 bis 100 °C liegt und/oder die Kopftemperatur der Destillationskolonne im Bereich von 25 bis 50°C.

Bevorzugt wird der Kopfabzug der Destillationsvorrichtung (z.B. E) in Kondensatoren verflüssigt, als HCN-Reinstrom (z.B. 11) abgeführt und bevorzugt teilweise als Rücklauf in die Destillationsvorrichtung (z.B. E) zurückgeführt. Bevorzugt liegt das Rücklaufverhältnis in Bereich von 1,5 bis 3,5; bevorzugt 2 bis 3.

Bevorzugt wird in der Destillationsvorrichtung (z.B. 11) ein Stabilisator, beispielsweise ausgewählt aus Schwefelsäure, Phosphorsäure oder Ameisensäure, zugegeben, um die Polymerisation von HCN und der Nitril-Nebenprodukte zu verhindern.

Typischerweise ist der HCN-Reinstrom (z.B. 11), welcher im Destillationsschritt v) erhalten wird, abgereichert an Nitrilen, insbesondere abgereichert an C2-C10-Nitrilen. Bevorzugt entweicht Acrylnitril (ACN) überwiegend über den Kopfstrom (z.B. 11) (HCN-Reinstrom) der Destillationsvorrichtung (z. B. E), während Acetonitril und andere C3-C10 Nitrile bevorzugt über den Nitril-haltigen, wässrigen Sumpfstrom (z.B. 12) aus der Destillationsvorrichtung (z.B. E) gelangen. Typischerweise erhöht sich die Nitril-Konzentration in der Destillationskolonne nach dem Anfahren zunehmend, bis sich ein Gleichgewicht der Ausschleusung und dem Kopfabzug einstellt.

Der HCN-Reinstrom (z.B. 11), welcher in Schritt v) erhalten wird, enthält 500 bis 5.000 ppm, bevorzugt 500 bis 4.490 ppm Acetonitril; 0 bis 1.000 ppm, bevorzugt 10 bis 800 ppm C3-Nitrile, insbesondere Acrylnitril und Propionitril, als Nitril-Nebenprodukt und 100 bis 7.000 ppm, bevorzugt 200 bis 6.000 ppm, Wasser, jeweils bezogen auf den gesamten HCN-Reinstrom.

Bevorzugt enthält der Nitril-haltige, wässrige Sumpfstrom (z.B. 12), welcher in Schritt v) erhalten wird, mehr als 99,0 Gew.-% Wasser und 10 bis 1.800 ppm, bevorzugt 50 bis 1.600 ppm, besonders bevorzugt 100 bis 1.500 ppm Acetonitril und 0 bis 1.000 ppm, bevorzugt 0 bis 800 ppm C3-Nitrile, insbesondere Acrylnitril und Propionitril, als Nitril-Nebenprodukt, jeweils bezogen auf den gesamten Nitril-haltigen, wässrigen Sumpfstrom.

Erfindungsgemäß erfolgt die Ausschleusung der im HCN-haltigen Prozessgas (z.B. 4) enthaltenen C2-C10 Nitril-Nebenprodukte aus dem Verfahren teilweise dadurch, dass der Nitril-haltige, wässrige Sumpfstrom (z.B. 12), welcher in Schritt v) erhalten wird, zumindest teilweise aus dem Verfahren ausgeschleust wird (z.B. über 13a) und/oder zumindest teilweise in die erste Absorptionsvorrichtung (z.B. B) in Schritt ii) (z.B. über (13c)) zurückgeführt wird. Im Fall der vollständigen oder teilweise Rückführung in die erste Absorptionsvorrichtung können die Nitril-Nebenprodukte über die wässrige Ammoniumsulfat-haltige Lösung (z.B. 6) und ggf. über das HCN-abgereicherte Armgas (z.B. 7) aus dem Verfahren ausgeschleust werden.

In einer bevorzugten Ausführungsform wird der Nitril-haltige, wässrige Sumpfstrom (z.B. 12), welcher in Schritt v) erhalten wird, zumindest teilweise aus dem Verfahren ausgeschleust, wobei der ausgeschleuste Strom (z.B. 13a) 10 bis 1.800 ppm, bevorzugt 50 bis 1.600 ppm, besonders bevorzugt 100 bis 1.500 ppm, C2-C10-Nitrile, insbesondere ausgewählt aus Acetonitril, Acrylnitril und Propionitril, bezogen auf den gesamten ausgeschleusten Strom, enthält.

In einer weiteren bevorzugten Ausführungsform wird der Nitril-haltige, wässrige Sumpfstrom (z.B. 12), welcher in Schritt v) erhalten wird, zumindest teilweise in die erste Absorptionsvorrichtung (z.B. B) in Schritt ii) zurückgeführt, wobei der in Schritt ii) zurückgeführt Strom (z.B. 13c), 10 bis 1.800 ppm, bevorzugt 50 bis 1.600 ppm, besonders bevorzugt 100 bis 1.500 ppm, C2-C10-Nitrile, insbesondere ausgewählt aus Acetonitril, Acrylnitril und Propionitril, bezogen auf den gesamten zurückgeführt Strom, enthält.

Weiterhin ist es bevorzugt, einen Teil des Nitril-haltigen, wässrige Sumpfstroms (z.B. 12), welcher in Schritt v) erhalten wird, zum Erwärmen der ersten wässrigen HCN-haltigen Lösung (z.B. 8) in der mindestens einen Wärmeaustauschapparatur zu verwenden und danach in die zweite Absorptionsvorrichtung (z.B. C) zurückzuführen (z.B. über 13b oder 9).

Bevorzugt wird der Nitril-haltige, wässriger Sumpfstrom (z.B. 12) in Behältern gepuffert und gleichmäßig, bevorzugt über die Wärmeaustauschapparatur (z.B. D), als wässriges Absorptionsmittel der zweiten Absorptionsvorrichtung (z.B. C) in Schritt iii) zugegeben. Aus den Pufferbehältern kann bevorzugt ein Seitenstrom zur Abwasserbehandlung abgezogen werden.

### Optionale Verfahrensschritte

Bevorzugt können die ausgeschleusten wässrigen Nitril-haltigen Ströme, insbesondere die wässrige Ammoniumsulfat-haltige Lösung (z.B. 6), welche in Schritt ii) erhalten wird, und/oder der Nitril-haltige, wässrige Sumpfstrom (z.B. 12), welcher in Schritt v) erhalten wird und welcher aus dem Verfahren ausgeschleust wird (z.B. 13a), weiter gereinigt werden, um den Gehalt an Blausäure und/oder Nitrilen zu verringern. Beispielsweise ist somit eine günstigere und umweltverträglichere Entsorgung oder eine Weiterverwendung der ausgeschleusten Stoffströme möglich. Dies kann insbesondere in den optionalen Aufarbeitungsteilen III und/oder IV erfolgen.

In einer bevorzugten Ausführungsform umfasst das Verfahren einen optionalen Aufarbeitungsteil III, wobei der Nitril-haltige, wässrige Sumpfstrom (z.B. 12), welcher in Schritt v) erhalten wird, zumindest teilweise in mindestens einer Stripp-Kolonne (z.B. F) mit einem Stripp-Gas (z.B. 17) behandelt wird, wobei die enthaltenen Nitril-Nebenprodukte, insbesondere ausgewählt aus C2-C10 Nitrilen, zumindest teilweise in Form des Stripp-Abgases (z.B. 18 b) entfernt werden, und wobei ein gereinigter wässriger Strom erhalten wird, der zumindest teilweise aus dem Verfahren ausgeschleust wird (z.B. 18a) und/oder zumindest teilweise in die erste Absorptionsvorrichtung (z.B. B) in Schritt ii) zurückgeführt wird (z.B. 18c). Bevorzugt kann der gereinigte wässriger Strom vollständig aus dem Verfahren ausgeschleust und als Abwasser entsorgt werden.

In einer bevorzugten Ausführungsform umfasst das Verfahren einen optionalen Aufarbeitungsteil III, wobei der ausgeschleuste Nitril-haltige, wässrige Sumpfstrom (z.B. 13a) in mindestens einer Stripp-Kolonne (z.B. F) behandelt wird.

Bevorzugt umfasst das Verfahren einen optionalen Aufarbeitungsteil IV, wobei in mindestens einem Schritt Blausäure und/oder Nitrile aus der wässrigen Ammoniumsulfat(AMSUL)-haltige Lösung (6) entfernt werden, und wobei in mindestens einem weiteren Schritt AMSUL aus der gereinigten wässrigen Lösung, z.B. als Feststoff, isoliert werden kann.

Bevorzugt erfolgt die Aufreinigung im optionalen Aufarbeitungsteil IV in der Weise, dass die AMSUL-haltige Lösung (z.B. 6) nach der Aufarbeitung sicher entsorgt und/oder als Düngemittel eingesetzt werden kann.

In einer bevorzugten Ausführungsform umfasst der optionalen Aufarbeitungsteil IV mindestens eine zweite Stripp-Kolonne (z.B. G), in der die AMSUL-haltige Lösung (z.B. 6) mit einem Stripp-Gas (z.B. 17), beispielsweise Wasserdampf, behandelt wird, wobei Blausäure und/oder C2-C10 Nitril-Nebenprodukte zumindest teilweise im Stripp-Abgas (z.B. 19b) entfernt werden, und wobei eine gereinigte AMSUL-Lösung (z.B. 19a) erhalten wird. Die gereinigte AMSUL-Lösung kann optional in eine Kristallisation (z.B. H) geführt werden, wobei kristallisiertes AMSUL (z.B. 20b) und eine Mutterlauge (z.B. 20a) erhalten werden. Das feste, kristallisierte AMSUL wird aus dem Verfahren ausgeschleust und kann bevorzugt als Düngemittel verwendet werden. Die erhaltene Mutterlauge kann optional in einer Entgiftung (z.B. H) mit einem Entgiftung-Reagenz (z.B. 21), das beispielsweise Kupfersalze und H₂O₂ enthält, behandelt werden, wobei eine entgiftete Mutterlauge (z.B. 22) erhalten wird, die problemlos als Abwasser entsorgt werden kann. Es ist zudem möglich, die gereinigte AMSUL-Lösung (z.B. 19a) aus dem Verfahren aus zu schleusen und als Düngemittel zu verwenden.

Vorteilhaft können ein oder mehrere Stabilisatoren in verschiedenen Stoffströmen des erfindungsgemäßen Verfahrens zugesetzt werden, um eine Polymerisation der C2-C10-Nitrile und der Blausäure zu verhindern oder zu reduzieren. Beispielsweise kann ein Stabilisator im Aufarbeitungsteil II, insbesondere bei der Destillation der zweiten wässrigen HCN-haltigen Lösung (10) in der Destillationsvorrichtung (E), zugegeben werden. Bevorzugt können organische Säuren, Mineralsäuren, Nichtmetalloxide und andere adäquat wirkende Stabilisatoren eingesetzt werden. Bevorzugt kommen organische Säuren, Mineralsäuren oder Nichtmetalloxide zum Einsatz. Besonders bevorzugt werden Schwefelsäure, Phosphorsäure, Essigsäure, Oxalsäure oder Schwefeldioxid eingesetzt.

### Variante A

In einer bevorzugten Ausführungsform der Erfindung (im Folgenden auch als Variante A bezeichnet) umfasst das erfindungsgemäße Verfahren zur Herstellung von HCN in Schritt i) die Umsetzung einer Methan-Quelle (z.B. 1), bevorzugt Erdgas, einer Ammoniak-Quelle (z.B. 2) und einer Sauerstoff-Quelle (z.B. 3) in einem Reaktor (z.B. A) in einem Reaktionsteil I unter Verwendung eines platinhaltigen Kontaktes (Andrussow-Verfahren).

Gemäß Variante A umfasst das erfindungsgemäße Verfahren zur Herstellung von HCN, dass der Nitril-haltige, wässrige Sumpfstrom (z.B. 12), welcher in Schritt v) erhalten wird, teilweise aus dem Verfahren ausgeschleust wird (z.B. 13a) und teilweise in die zweite Absorptionsvorrichtung (C) in Schritt iii) zurückgeführt wird (z.B. über 13b und/ 9). Bevorzugt wird dieser zurückgeführte Nitril-haltige, wässrige Sumpfstrom (z.B. 13b) über die mindestens eine Wärmeaustauschapparatur (z.B. D) geführt, um die erste wässrige HCN-haltige Lösung (z.B. 8), welche in Schritt iii) erhalten wird, zu erwärmen.

Gemäß Variante A enthält der ausgeschleuste Nitril-haltige, wässrige Sumpfstrom (z.B. 13a) 10 bis 1.800 ppm, bevorzugt 50 bis 1.600 ppm, besonders bevorzugt 100 bis 1.500 ppm, C2-C10-Nitrile, insbesondere ausgewählt aus Acetonitril, Acrylnitril und Propionitril, bezogen auf den gesamten ausgeschleusten Strom.

Gemäß Variante A des erfindungsgemäßen Verfahrens wird als saures, wässriges Absorptionsmittel in Schritt ii) eine wässrige Mischung mindestens einer Brönsted-Säure, insbesondere Schwefelsäure, eingesetzt, welche als Frischsäure (z.B. 16) in die erste Absorptionsvorrichtung (z.B. B) zugegeben wird.

Bevorzugt wird in Variante A des erfindungsgemäßen Verfahrens die AMSUL-haltige Lösung (z.B. 6) im optionalen Aufarbeitungsteil IV, wie oben beschrieben, behandelt, wobei kristallisiertes AMSUL aus dem Verfahren ausgeschleust und bevorzugt als Düngemittel verwendet wird.

### Variante B

In einer bevorzugten Ausführungsform der Erfindung (im Folgenden auch als Variante B bezeichnet) umfasst das erfindungsgemäße Verfahren zur Herstellung von HCN in Schritt i) die Umsetzung einer Methan-Quelle (z.B. 1), bevorzugt Erdgas oder eines methanreiches Gases aus einem Steamcracker, und einer Ammoniak-Quelle (z.B. 2) in einem Reaktor (z.B. A) in einem Reaktionsteil I unter Verwendung eines platinhaltigen Kontaktes (BMA-Verfahren).

Gemäß Variante B umfasst das erfindungsgemäße Verfahren zur Herstellung von HCN, dass der Nitril-haltige, wässrige Sumpfstrom (z.B. 12), welcher in Schritt v) erhalten wird, teilweise in die erste Absorptionsvorrichtung (z.B. B) in Schritt ii) zurückgeführt wird, und teilweise in die zweite Absorptionsvorrichtung (C) in Schritt iii) zurückgeführt wird (z.B. über 13b und 9). Bevorzugt wird dieser zurückgeführte Nitril-haltige, wässrige Sumpfstrom (z.B. 13b) über die mindestens eine Wärmeaustauschapparatur (z.B. D) geführt, um die erste wässrige HCN-haltige Lösung (z.B. 8), welche in Schritt iii) erhalten wird, zu erwärmen.

Gemäß Variante B enthält der in Schritt ii) zurückgeführt Strom (z.B. 13c) bevorzugt 10 bis 1.800 ppm, bevorzugt 50 bis 1.600 ppm, besonders bevorzugt 100 bis 1.500 ppm, C2-C10-Nitrile, insbesondere ausgewählt aus Acetonitril, Acrylnitril und Propionitril, bezogen auf den gesamten zurückgeführt Strom.

Gemäß Variante B des erfindungsgemäßen Verfahrens wird als saures, wässriges Absorptionsmittel in Schritt ii) der zurückgeführter Nitril-haltige, wässrige Sumpfstrom (z.B. 13c über 15) verwendet, dem bevorzugt Frischsäure (z.B. 14), bevorzugt Schwefelsäure, zugegeben wird. Bevorzugt kann in Variante B die Zugabe von Frischsäure direkt in die erste Absorptionsvorrichtung (B) entfallen.

Bevorzugt wird in Variante B des erfindungsgemäßen Verfahrens eine AMSUL-haltige Lösung (z.B. 6) oder alternativ eine in mindestens einer zweiten Stripp-Kolonne (z.B. G) gereinigte AMSUL-haltige Lösung (z.B. 19a) aus dem Verfahren ausgeschleust und als Düngemittel verwendet.

In einer bevorzugten Ausführungsform der Variante B kann das HCN-abgereicherte Armgas (z.B. 7), das in Schritt iii) erhalten wird, als Stripp-Gas der optionalen zweiten Stripp-Kolonne (z.B. G) verwendet werden.

### Herstellung von Methacrylsäure (MAS) und/oder Alkylmethacrylaten

Weiterhin betrifft die Erfindung ein Verfahren zur Herstellung von Methacrylsäure (MAS) und/oder Alkylmethacrylaten, insbesondere Methylmethacrylat (MMA), in einem ACH-Sulfo-Prozess unter Verwendung erfindungsgemäß hergestellter Blausäure, wobei das Verfahren die Umsetzung der erfindungsgemäß hergestellten Blausäure mit Aceton, in Gegenwart eines basischen Katalysators zu Acetoncyanhydrin (ACH) in einer ersten Reaktionsstufe, die Umsetzung von Acetoncyanhydrin (ACH) und Schwefelsäure zu Methacrylamid (MASA) in einer zweiten Reaktionsstufe (Amidierung und Konvertierung) sowie die anschließende Hydrolyse bzw. Veresterung von Methacrylamid (MASA) mit Wasser bzw. mit Alkohol und Wasser, bevorzugt Methanol und Wasser, unter Bildung von Methacrylsäure bzw. Alkylmethacrylat in einer dritten Reaktionsstufe, umfasst. Typischerweise kann sich die Aufarbeitung der erhaltenen Reaktionsmischung zur Isolierung von MAS und/oder Alkylmethacrylaten, insbesondere MMA, anschließen.

In diesem Zusammenhang betrifft die vorliegende Erfindung ein Verfahren zur Herstellung von Methacrylsäure und/oder Alkylmethacrylaten, insbesondere Methylmethacrylat (MMA), umfassend die folgenden Schritte:
xi. Herstellung von Blausäure in einem erfindungsgemäßen Verfahren wie oben beschrieben;
xii. Umsetzung der Blausäure erhalten in Schritt xi) und Aceton in Gegenwart eines basischen Katalysators in einer ersten Reaktionsstufe (Synthese von ACH), wobei eine erste Reaktionsmischung enthaltend Acetoncyanhydrin (ACH) erhalten wird;
xiii. Aufarbeitung der ersten Reaktionsmischung, enthaltend Acetoncyanhydrin (ACH);
xiv. Umsetzung von Acetoncyanhydrin und Schwefelsäure in einem oder mehreren Reaktoren I in einer zweiten Reaktionsstufe (Amidierung) bei einer Amidierungstemperatur, bevorzugt im Bereich von 85°C bis 130°, wobei eine zweite Reaktionsmischung, enthaltend Sulfoxyisobuttersäureamid und Methacrylamid, erhalten wird;
xv. Konvertieren der zweiten Reaktionsmischung, umfassend Erhitzen auf eine Konvertierungstemperatur, bevorzugt im Bereich von 130°C bis 200°C, in einem oder mehreren Reaktoren II in einer dritten Reaktionsstufe (Konvertierung), wobei eine dritte Reaktionsmischung, enthaltend überwiegend Methacrylamid (MASA) und Schwefelsäure, erhalten wird;
xvi. Umsetzung der dritten Reaktionsmischung mit Wasser und optional Alkohol, bevorzugt Wasser und optional Methanol, in einem oder mehreren Reaktoren III in einer vierten Reaktionsstufe (Hydrolyse oder Veresterung), wobei eine vierte Reaktionsmischung, enthaltend Methacrylsäure und/oder Alkylmethacrylat, bevorzugt Methylmethacrylat, erhalten wird;
xvii. optional Aufarbeitung der vierten Reaktionsmischung zur Isolierung von Methacrylsäure und / oder Alkylmethacrylaten, insbesondere Methylmethacrylat (MMA).

Weitere Details und Ausführungsformen zur Herstellung von Methacrylsäure und/oder Alkylmethacrylaten, insbesondere Methylmethacrylat (MMA), nach dem ACH-Sulfo-Verfahren sind dem Fachmann bekannt und beispielsweise in den Internationalen Patentanmeldungen PCT/EP2021/078866 und PCT/EP2021/077488 beschrieben.

### Beschreibung der Figuren

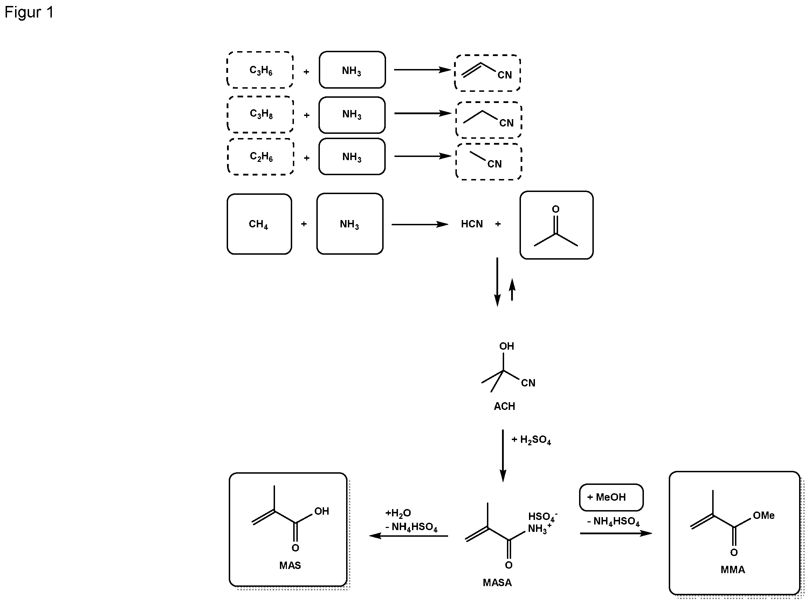

Figur 1 beschreibt das Reaktionsnetzwerk der Bildung von Blausäure (HCN) und deren anschließende Umsetzung zu Methacrylsäure und/oder Methylmethacrylat nach dem ACH-Sulfo-Verfahren. Ausgehend von Methan (CH₄) und Ammoniak (NH₃) kann über das BMA-Verfahren (Blausäure aus Methan und Ammoniak) oder über das Andrussow-Verfahren Blausäure hergestellt werden. Die höheren Alkane, Ethan, Propan und Propen, bilden hierbei störende Nitril-Nebenprodukte (Acetonitril ACN, Acrylnitril ACRN und Propionitril PN).

Im nächsten Schritt wird ausgehend von Aceton und Blausäure, unter Zugabe eines basischen Katalysators Acetoncyanhydrin (ACH) hergestellt. Acetoncyanhydrin (ACH) wird dann mit Schwefelsäure über mehrere Zwischenstufen zu Methacrylamid-hydrogensulfat (MASA·H₂SO₄) umgesetzt. Nachfolgend kann Methacrylamid-hydrogensulfat (MASA·H₂SO₄) durch Hydrolyse zu Methacrylsäure (MAS) oder durch Veresterung mit Methanol (MeOH) zu Methylmethacrylat (MMA) umgewandelt werden.

Die Abkürzungen in Figur 1 haben die folgenden Bedeutungen:
- ACH: Acetoncyanhydrin
- ACN: Acetonitril
- ACRN: Acrylnitril
- MAS: Methacrylsäure
- MMA: Methylmethacrylat
- PN: Propionitril
- MASA: Methacrylsäureamid / Methacrylamid;
- MAS: Methacrylsäure;
- MMA: Methylmethacrylat;

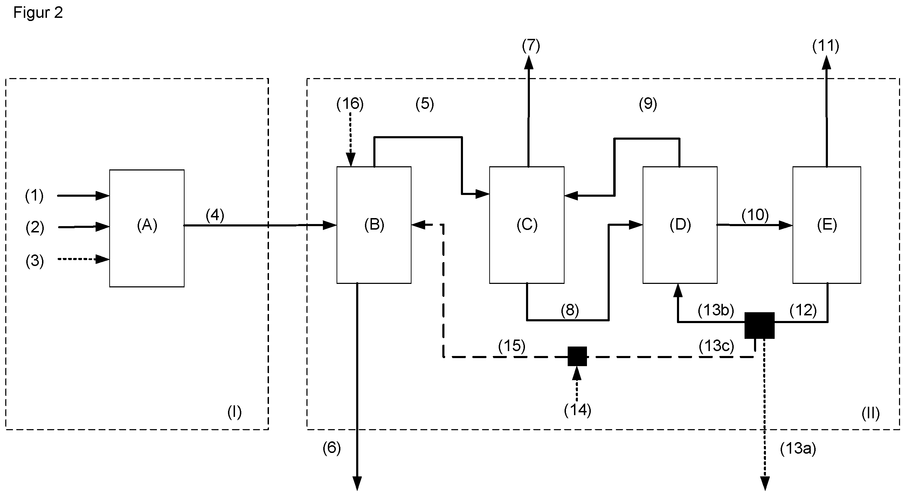

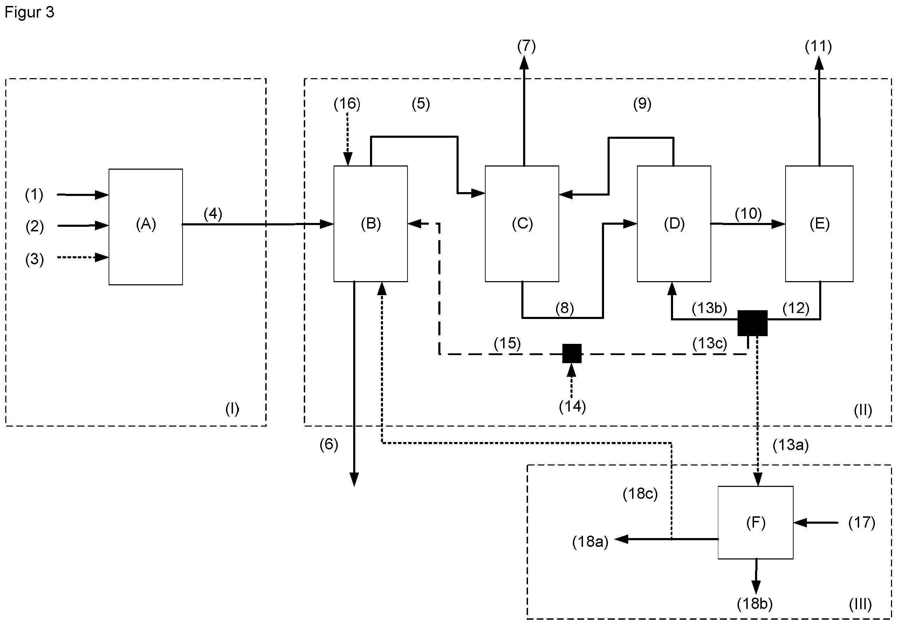

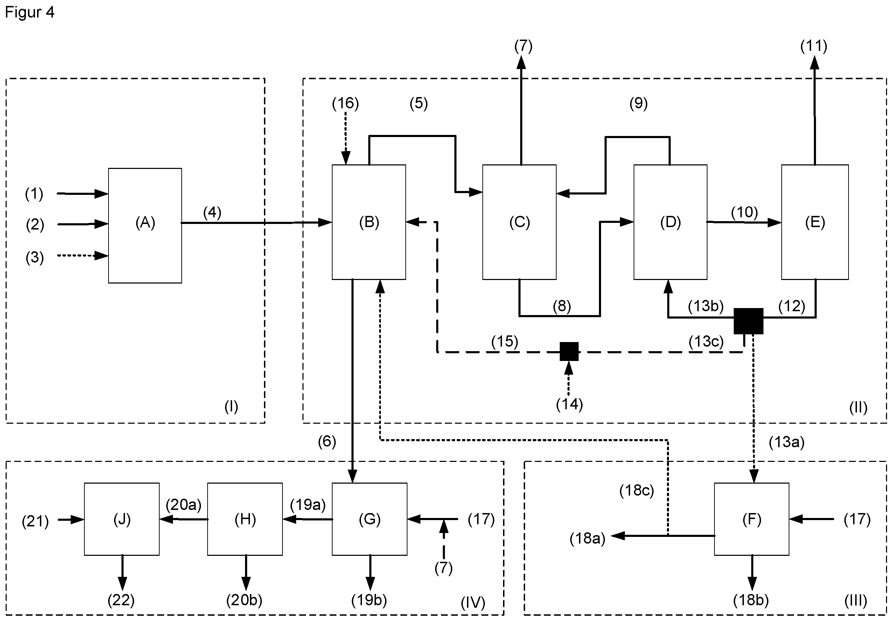

Figur 2 zeigt ein Fließschema einer bevorzugten Ausführungsform des erfindungsgemäßen Verfahrens. Die Figuren 3 und 4 zeigen Fließschema von weiteren bevorzugten Ausführungsformen des erfindungsgemäßen Verfahrens umfassend zusätzliche optionale Verfahrensschritte, nämlich optionale Aufarbeitungsteile III und IV. Es sei angemerkt, dass weitere dem Fachmann bekannte Komponenten bei der Durchführung des erfindungsgemäßen Verfahrens eingesetzt werden können. So weist beispielsweise in der Regel jede der aufgeführten Kolonnen einen Kondensator auf. Zudem ist nicht jede mögliche oder bevorzugte Ausführungsform in den Figuren berücksichtigt. Die Position der Zuleitungen gibt in der Regel nicht deren reale Lage an, sondern verdeutlicht nur die topologische Anordnung der entsprechenden Verfahrensschritte.

### Bezugszeichenliste

In den Figuren 2 bis 4 haben die Bezugszeichen die folgenden Bedeutungen:
Apparate

| | |
|---|---|
| (A) | Reaktor |
| (B) | Erste Absorptionsvorrichtung |
| (C) | Zweite Absorptionsvorrichtung |
| (D) | Wärmeaustauschapparatur |
| (E) | Destillationsvorrichtung |
| (F) | Optionale erste Stripp-Kolonne |
| (G) | Optionale zweite Stripp-Kolonne |
| (H) | Optionale Kristallisation |
| (J) | Optionale Entgiftung |

Stoffströme

| | |
|---|---|
| (1) | Methan-Quelle |
| (2) | Ammoniak-Quelle |
| (3) | Sauerstoff-Quelle (z.B. Luft) |
| (4) | HCN-haltiges Prozessgas |
| (5) | Ammoniak-abgereichertes Prozessgas |
| (6) | Wässriger Ammoniumsulfat-haltiger Strom |
| (7) | HCN-abgereichertes Armgas |
| (8) | Erste wässrige HCN-haltige Lösung |
| (9) | Gekühlter Nitril-haltiger, wässriger Sumpfstrom (12) |
| (10) | Zweite wässrige HCN-haltige Lösung (vorgewärmter Destillations-Feedstrom) |
| (11) | HCN-Reinstrom (Nitril-abgereicherter HCN-Reinstrom) |
| (12) | Nitril-haltiger, wässriger Sumpfstrom |
| (13a) | Ausgeschleuster Nitril-haltiger, wässriger Sumpfstrom (12) |
| (13b) | Zurückgeführter Nitril-haltiger, wässriger Sumpfstrom (12) zu (D) |
| (13c) | Zurückgeführter Nitril-haltiger, wässriger Sumpfstrom (12) zu (B) |
| (14) | Optionale Schwefelsäure-Zugabe |
| (15) | Zurückgeführter Nitril-haltiger, wässriger Strom zu (B) |
| (16) | Optionale Schwefelsäure-Zugabe |
| (17) | Stripp-Gas |
| (18a) | Gereinigter, Nitril-haltiger, wässriger Sumpfstrom zur Ausschleusung |
| (18b) | Abgas der ersten Stripp-Kolonne |
| (18c) | Gereinigter, Nitril-haltiger, wässriger Sumpfstrom zur Rückführung |
| (19a) | Gereinigte AMSUL-Lösung |
| (19b) | Abgas der zweiten Stripp-Kolonne |
| (20a) | Mutterlauge der Kristallisation |
| (20b) | Kristallisiertes AMSUL |
| (21) | Entgiftungs-Reagenz |
| (22) | Entgiftete Mutterlauge |

Der in Figur 2 schematisch dargestellte Anlagenverbund umfasst den Reaktor A (Blausäuresynthese-Reaktor), in den eine Methan-Quelle 1, eine Ammoniak-Quelle 2 und optional eine Sauerstoffquelle 3 gespeist werden. Dieser Reaktor wird gefolgt von einer ersten Absorptionsvorrichtung B, in der nicht umgesetztes Ammoniak aus dem HCN-haltigen Prozessgas 4 entfernt wird, wobei ein Ammoniak-abgereichertes Prozessgases 5 und eine wässrige Ammoniumsulfat(AMSUL)-haltige Lösung erhalten werden. Typischerweise wird die wässrige AMSUL-haltige Lösung als Strom 6 aus dem Verfahren ausgeschleust. Das in Absorptionsschritt ii) eingesetzte saure, wässrige Absorptionsmittel ist beispielweise Schwefelsäure und kann über die die Schwefelsäurequellen 16 und/oder 14, bevorzugt über 14 oder 16, erfolgen.

Aus dem Ammoniak-abgereicherten Prozessgas 5 wird in einer anschließenden zweiten Absorptionsvorrichtung C Blausäure absorbiert, wobei als wässriges Absorptionsmittel 9 der gekühlte Nitril-haltige, wässriger Sumpfstrom aus der Destillation E verwendet wird. In der zweiten Absorptionsvorrichtung C wird eine erste wässrige HCN-haltige Lösung 8 und ein HCN-abgereichertes Armgas 7 erhalten. Die erste wässrige HCN-haltige Lösung 8 wird nach Erwärmung in der Wärmeaustauschappartur D einer Destillationsvorrichtung E als zweite wässrige HCN-haltige Lösung 10 zugeleitet. In der Destillationsvorrichtung E werden als Kopfprodukt ein HCN-Reinstrom 11, welcher an Nitril abgereichert ist, und ein Nitril-haltiger, wässriger Sumpfstrom 12 erhalten.

Der Nitril-haltige, wässrige Sumpfstrom 12 wird teilweise als Strom 13b in die Wärmeaustauschapparatur D zurückgeführt und als Wärmemedium für das Erwärmen der ersten wässrigen HCN-haltigen Lösung 8 verwendet.

Der Nitril-haltige, wässrige Sumpfstrom 12 kann teilweise als Strom 13c in die erste Absorptionsvorrichtung B zurückgeführt und dort als saures, wässriges Absorptionsmittel verwendet werden, wobei eine Schwefelsäure-Quelle 14 zugegeben wird. Alternativ kann der Nitril-haltige, wässrige Sumpfstrom 12 teilweise als Strom 13a aus dem Verfahren ausgeschleust werden, wobei dann in der ersten Absorptionsvorrichtung B eine Schwefelsäure-Quelle 16 als saures, wässriges Absorptionsmittel zugeführt wird.

Figur 3 zeigt den optionalen Aufarbeitungsteil III umfassend die Stripp-Kolonne F zur weiteren Aufarbeitung des wässrigen Sumpfstroms 13a der Destillation E. Der ausgeschleuste Nitril-haltige, wässrige Sumpfstrom 13a wird in der ersten Stripp-Kolonne F mit einem Stripp-Gas 17, beispielsweise Wasserdampf, behandelt, wobei Blausäure und/oder die enthaltenen C2-C10 Nitril-Nebenprodukte zumindest teilweise im Stripp-Abgas 18b entfernt werden. Der so gereinigte wässriger Strom 18a wird aus dem Verfahren ausgeschleust und als Abwasser entsorgt und kann optional zumindest teilweise als 18c in die erste Absorptionsvorrichtung B zurückgeführt werden.

Figur 4 zeigt den optionalen Aufarbeitungsteil IV, wobei die AMSUL-haltige Lösung 6 in einer zweiten Stripp-Kolonne G mit einem Stripp-Gas 17, beispielsweise Wasserdampf, behandelt wird, wobei Blausäure und/oder C2-C10 Nitril-Nebenprodukte zumindest teilweise im Stripp-Abgas 19b entfernt werden, und wobei eine gereinigte AMSUL-Lösung 19a erhalten wird. Optional kann das HCN-abgereicherte Armgas 7, das in Schritt iii) erhalten wird, als Stripp-Gas der optionalen zweiten Stripp-Kolonne G verwendet werden. Die gereinigte AMSUL-Lösung wird in die Kristallisation H geführt, wobei kristallisiertes AMSUL 20b und eine Mutterlauge 20a erhalten werden. Das feste, kristallisierte AMSUL wird aus dem Verfahren ausgeschleust und kann bevorzugt als Düngemittel verwendet werden. Die erhaltene Mutterlauge 20a wird in einer Entgiftung H mit dem Entgiftung-Reagenz 21, das beispielsweise Kupfersalze und H₂O₂ enthält, behandelt, wobei eine entgiftete Mutterlauge 22 erhalten wird, die problemlos als Abwasser entsorgt werden kann.

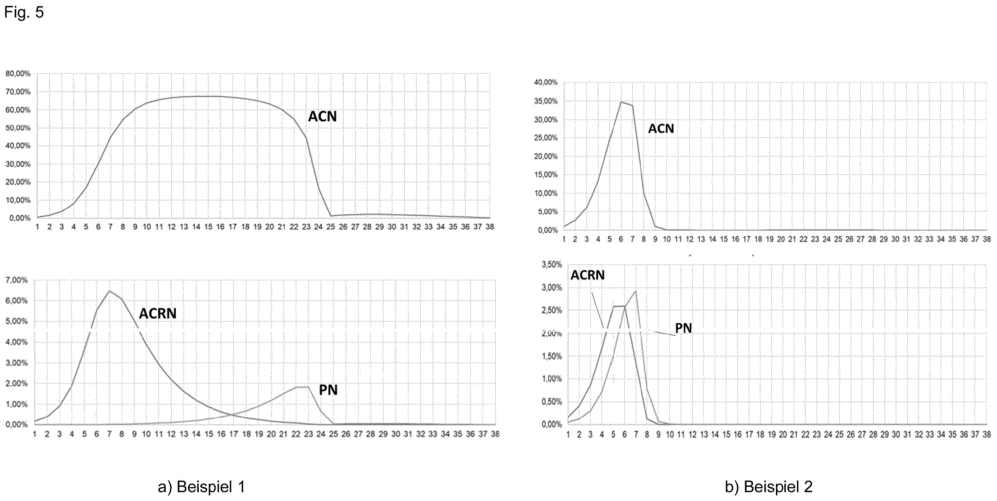

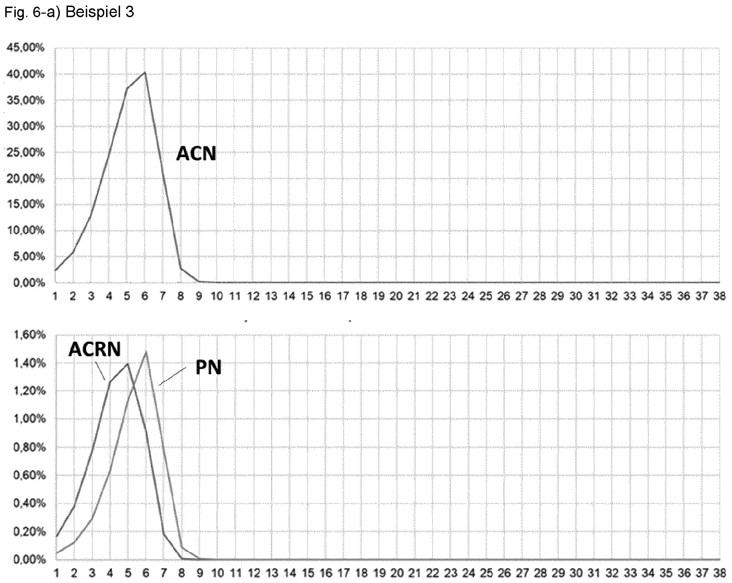

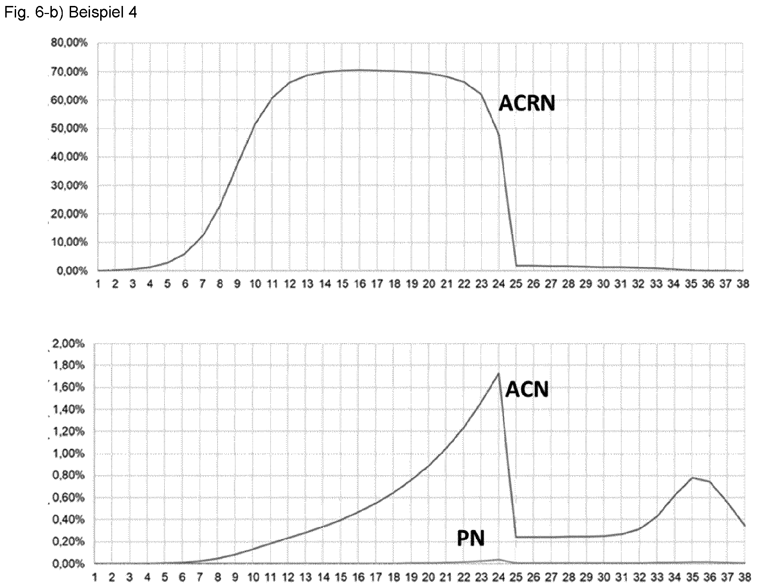

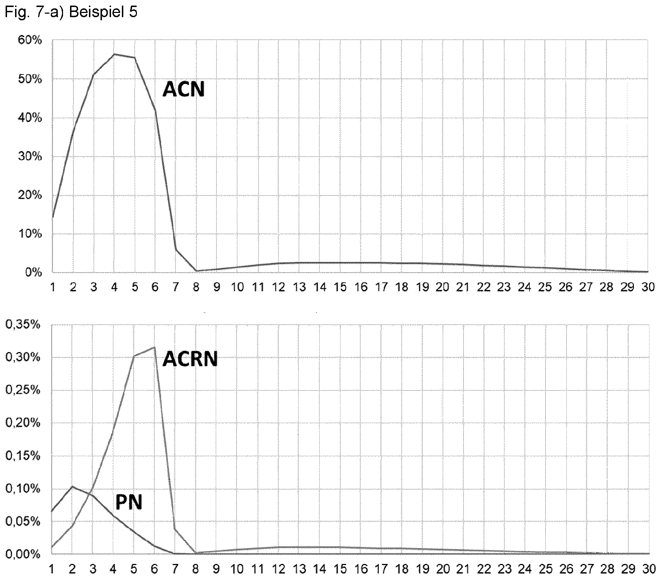

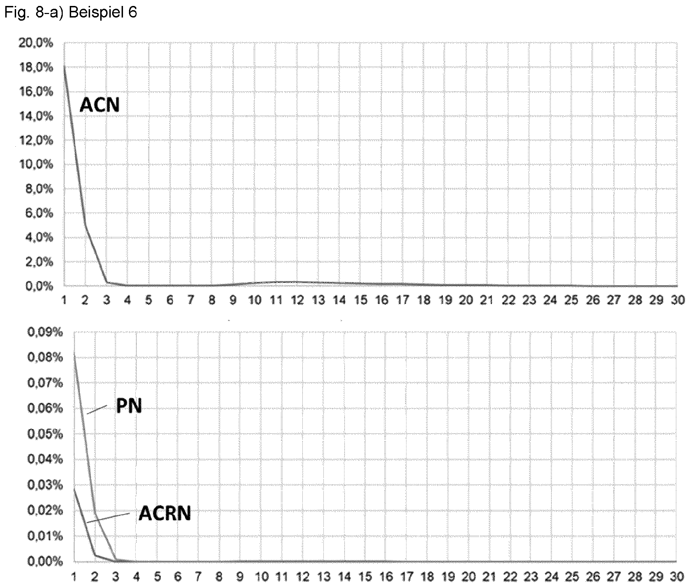

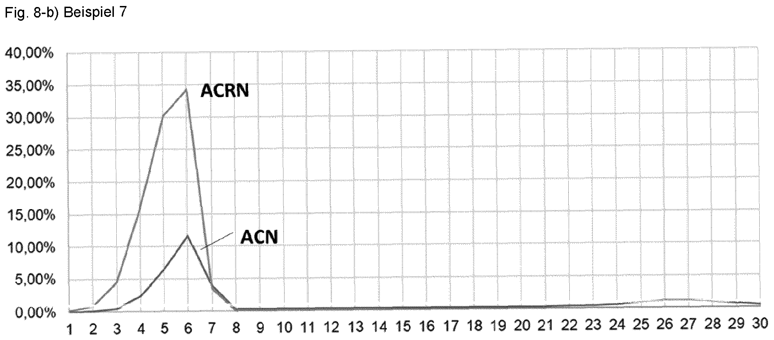

Die Figuren 5 bis 8 zeigen den Anteil an ACN, ACRN und PN in der wässrigen Phase der Destillationskolonne (E) in Abhängigkeit der Nummer der theoretischen Stufe der Destillation für die Beispiele 1 bis 7. Die x-Achse gibt die Nummer der theoretischen Stufe der Destillationskolonne (E) an, wobei 1 den Kopf und 38 den Sumpf der Kolonne E darstellt. Die y-Achse gibt die Konzentration des betreffenden Nitrils in der wässrigen Phase in Gew.-% an. Figur 5 bezieht sich auf die Ergebnisse gemäß den Beispielen 1 (Fig. 5a) und 2 (Fig. 5b), Figur 6 bezieht sich auf die Ergebnisse gemäß den Beispielen 3 (Fig. 6a) und 4 (Fig. 6b), Figur 7 bezieht sich auf die Ergebnisse gemäß Beispiel 5, Figur 8 bezieht sich auf die Ergebnisse gemäß den Beispielen 6 (Fig. 8a) und 7 (Fig. 8b).

Figur 9 zeigt das Flüssig-Flüssig-Gleichgewichtsdiagramm (LLE) einer ternären Mischung aus ACRN, HCN und Wasser am Siedepunkt der jeweiligen Zusammensetzung, wobei die Masseanteile der Komponenten aufgetragen sind. Das Flüssig-Flüssig-Gleichgewichtsdiagramm wurde durch Simulation basierend auf Parametern der binären Systeme und auf experimentellen Daten erstellt. Es wird verdeutlicht, dass Mischungen mit mehr als 10 Gew.-% ACRN und mit Wassergehalten über 50 Gew.-% in zwei Phasen zerfallen.

Die Erfindung wird anhand der folgenden experimentellen Beispiele näher erläutert, wobei die Erfindung nicht auf die Beispiele beschränkt anzusehen ist.

### Beispiele

Es wurden die folgenden Beispiele gemäß dem Prozess-Fließschema nach Figur 2 durchgeführt, wobei die Verteilung der Nitril-Nebenprodukte in Kopf- und Sumpf-Strom der Destillationskolonne (E) durch Einstellen der Destillationsparameter gemäß Tabelle 1 variiert wurde:

**Tabelle 1: Zusammenfassung Beispiele**

| Beispiel | Verfahren Reaktor A | Variante | T1 [°C] | T2 [°C] | H [kW] | K [kW] | Ausschleusung Nitrile |
|---|---|---|---|---|---|---|---|
| 1 | Andrussow | A | 49 | 66 | 2.760 | -1.593 | kombiniert über (13a), (6), (7) und (11) |
| 2 | Andrussow | A | 60 | 87 | 2.790 | -1.604 | kombiniert über (13a), (6), (7) und (11) |
| 3* | Andrussow | A | 65 | 87 | 2.805 | -1.619 | hauptsächlich über (11) und (6) |
| 4* | Andrussow | A | 31 | 60 | 2.753 | -1.584 | hauptsächlich über (13a), (7), und (6) |
| 5 | BMA | B | 68 | 96 | 4.793 | -1.879 | kombiniert über (6), (7) und (11) |
| 6* | BMA | B | 77 | 100 | 4.709 | -1.729 | hauptsächlich über (11) |
| 7* | BMA | B | 55 | 75 | 4.686 | -1.827 | hauptsächlich über (6) und (7) |

| | | | | | | | |
|---|---|---|---|---|---|---|---|
| *Vergleichsbeispiel T1: Temperatur in theoretischer Stufe 7 T2: Temperatur in theoretischer Stufe 15 H: Heizleistung Kolonne K: Kondensationsleistung Kolonne | | | | | | | |

Die Ergebnisse beruhen auf Simulationsergebnissen, wobei die stationären Blausäure- und Nitril-Konzentrationen durch HPLC-Analyse repräsentativer Proben, die aus einer kontinuierlich betriebenen technischen Produktionsanlage entnommen wurden, bestätigt wurden.

### Durchführung Beispiele 1-4:

In den Beispielen 1 bis 4 wurde das Verfahren gemäß Variante A (Andrussow-Prozess) wie in Figur 2 dargestellt und wie oben beschrieben durchgeführt. Im Reaktionsteil I umfassend Reaktor (A) erfolgte die Umsetzung einer Methan-Quelle (1), einer Ammoniak-Quelle (2) und einer Sauerstoff-Quelle (3) in Reaktor (A) unter Verwendung eines platinhaltigen Kontaktes, wobei ein HCN-haltiges Prozessgas (4) erhalten wurde. Es wurden die folgenden Edukt-Ströme eingesetzt: Methan-Quelle (1) (gasförmig) 1667 kg/h, Ammoniak-Quelle (2) (gasförmig) 1847 kg/h, 8505 kg/h Luft (gasförmig) und 1328 kg/h Sauerstoff (gasförmig) als Sauerstoff-Quelle (3). Die Zusammensetzung der Methan-Quelle (1) war wie folgt:
96,0 Vol.-% (92,43 Gew.-%) Methan (CH4);
1,4 Vol.-% (2,53 Gew.-%) Ethan (C2H6);
0,5 Vol.-% (1,32 Gew.-%) Propan (C3H8)
0,2 Vol.-% (0,53 Gew.-%) Kohlendioxid (CO2);
1,9 Vol.-% (3,19 Gew.-%) Stickstoff (N2).

Es wurde ein HCN-haltiges Prozessgas (4) nach Reaktor (A) erhalten, welches 9.8 Vol.-% HCN und Nitril-Konzentrationen wie in den Tabellen 3-6 angegeben aufwies.

Der Nitril-haltige, wässrige Sumpfstrom (12) wurde in die Ströme (13b) und (13a) aufgeteilt und somit zum einen über die Wärmeaustauschapparatur D in die zweite Absorptionsvorrichtung C als wässriges Absorptionsmittel (9) zurückgeführt und zum anderen aus dem Verfahren ausgeschleust. In der ersten Absorptionsvorrichtung (B) wurde als saures, wässriges Absorptionsmittel wässrige Schwefelsäure über (16) zudosiert, so dass die Ammoniumsulfat(AMSUL)-haltige Lösung (6), welche aus dem Verfahren ausgeschleust wurde, einen pH-Wert von etwa 3,5 aufwies.

Die wässrige AMSUL-haltige Lösung (6), welche in der ersten Absorptionsvorrichtung (B) erhalten wurde, wies Nitril-Konzentrationen wie in den Tabellen 3 bis 6 angegeben auf.

### Durchführung Beispiele 5-7:

In den Beispielen 5 bis 7 wurde das Verfahren gemäß Variante B (BMA-Prozess) wie in Figur 2 dargestellt und wie oben beschrieben durchgeführt. Im Reaktionsteil I umfassend Reaktor (A) erfolgte die Umsetzung einer Methan-Quelle (1) und einer Ammoniak-Quelle (2) in Reaktor (A) unter Verwendung eines platinhaltigen Kontaktes, wobei ein HCN-haltiges Prozessgas (4) erhalten wurde.

Es wurden die folgenden Edukt-Ströme eingesetzt: Methan-Quelle (1) (gasförmig) 3815 kg/h, und Ammoniak-Quelle (2) (gasförmig) 4577 kg/h. Die Zusammensetzung der Methan-Quelle (1) war wie folgt:
96,47 Vol.-% (98,00 Gew.-%) Methan (CH4);
0,26 Vol.-% (0,50 Gew.-%) Ethan (C2H6);
0,00 Vol.-% Propan (C3H8)
2,61 Vol.-% (0,33 Gew.-%) Wasserstoff (H2);
0,66 Vol.-% (1,17 Gew.-%) Stickstoff (N2).

Es wurde ein HCN-haltige Prozessgas (4) nach Reaktor (A) erhalten, welches 22,7 Vol.-% HCN und Nitril-Konzentrationen wie in den Tabellen 7 bis 9 angegeben aufwies.

Der Nitril-haltige, wässrige Sumpfstrom (12) wurde in die Ströme (13b) und (13c) aufgeteilt und somit zum einen über die Wärmeaustauschapparatur D in die zweite Absorptionsvorrichtung C als wässriges Absorptionsmittel (9) zurückgeführt und zum anderen in die erste Absorptionsvorrichtung als saures, wässrigen Absorptionsmittel (15) zurückgeführt. Hierbei wurden Wasser und Schwefelsäure über (14) zudosiert, so dass die AMSUL-haltige Lösung (6), welche aus dem Verfahren ausgeschleust wurde, einen pH-Wert von etwa 3,5 aufwies.

Die wässrige Ammoniumsulfat-haltige Lösung (6), welche in der ersten Absorptionsvorrichtung (B) erhalten wurde, wies Nitril-Konzentrationen wie in den Tabellen 7 bis 9 angegeben auf.

**Tabelle 2: Überblick Ergebnisse Beispiele 1-7**

| Beispiel | | 1 | 2 | 3* | 4* | 5 | 6* | 7* |
|---|---|---|---|---|---|---|---|---|
| Variante | | A | A | A | A | B | B | B |
| Gesamt (11) | kg/h | 1.727 | 1.749 | 1.749 | 1714 | 5.765 | 5.805 | 5.735 |
| HCN-Anteil in (11) | Gew.-% | 99,4 | 98,9 | 98,2 | 99,8 | 99,3 | 98,5 | 99,8 |
| Wasser-Anteil in (11) | Gew.-% | 0,2 | 0,5 | 0,8 | 0,04 | 0,5 | 1,1 | 0,1 |
| HCN-Anteil in (12) | ppm | 0 | 0 | 0 | 378 | 0 | 0 | 65 |
| Nitrile (gesamt) in (12) | ppm | 1.420 | 10 | 10 | 1.948 | 1.127 | 10 | 1.903 |
| Nitrile (gesamt) in (11) | ppm | 2.738 | 4.460 | 8.955 | 356 | 1.816 | 4.123 | 17 |
| Gesamt (13a) | kg/h | 1.055 | 1.025 | 1.044 | 1.033 | nv | nv | nv |
| Gesamt (13c) | kg/h | nv | nv | nv | nv | 170 | 368 | 424 |

| | | | | | | | | |
|---|---|---|---|---|---|---|---|---|
| *Vergleichsbeispiel, nv=nicht vorhanden | | | | | | | | |

**Tabelle 3: Bilanz Nitrile Beispiel 1 (erfindungsgemäß)**

| | | Armgas (7) | Ausgeschleuster Sumpf-strom (13a) | AMSUL haltiger Strom (6) | HCN-Reinstrom (11) | HCN-haltiges Prozessgas (4) |
|---|---|---|---|---|---|---|
| ACN | kg/h | 9,22 | 1,47 | 1,17 | 3,67 | 15,52 |
| | Gew.-% | 0,1143 | 0,1387 | 0,0347 | 0,2127 | 0,12 |
| | % von (4) | 59,41 | 9,47 | 7,53 | 23,63 | 100 |
| ACRN | kg/h | 0 | 0 | 0 | 1,06 | 1,06 |
| | ppm | 0 | 0 | 1 | 611 | 80 |
| | % von (4) | 0 | 0 | 0 | 100 | 100 |
| PN | kg/h | 0,23 | 0,03 | 0,01 | 0 | 0,28 |
| | ppm | 28 | 33 | 3 | 0 | 21 |
| | % von (4) | 82,14 | 10,71 | 3,57 | 0 | 100 |

**Tabelle 4: Bilanz Nitrile Beispiel 2 (erfindungsgemäß)**

| | | (7) | (13a) | (6) | (11) | (4) |
|---|---|---|---|---|---|---|
| ACN | kg/h | 0,03 | 0 | 0,53 | 6,33 | 6,9 |
| | Gew.-% | 0,0004 | 0 | 0,02 | 0,37 | 0,05 |
| | % von (4) | 0,43 | 0 | 7,68 | 91,73 | 100 |
| ACRN | kg/h | 0 | 0 | 0 | 1,06 | 1,06 |
| | ppm | 0 | 0 | 0 | 608 | 80 |
| | % von (4) | 0 | 0 | 0 | 100 | 100 |
| PN | kg/h | 0 | 0 | 0,01 | 0,26 | 0,28 |
| | ppm | 0 | 0 | 3 | 152 | 21 |
| | % von (4) | 0 | 0 | 3,57 | 92,86 | 100 |
| HCN | kg/h | 2,28 | 0,0 | 0,0 | 1.716 | 1.726 |
| | Gew.-% | - | - | - | 98,91 | 13,05 |
| | ppm | 281 | 0 | 0 | - | - |

**Tabelle 5: Bilanz Nitrile Beispiel 3 (Vergleichsbeispiel)**

| | | (7) | (13a) | (6) | (11) | (4) |
|---|---|---|---|---|---|---|
| ACN | kg/h | 0,06 | 0,01 | 1,17 | 14,28 | 15,52 |
| | Gew.-% | 0,0008 | 0,001 | 0,03 | 0,82 | 0,12 |
| | % von (4) | 0,4 | 0,06 | 7,5 | 92,0 | 100 |
| ACRN | kg/h | 0 | 0 | 0 | 1,06 | 1,06 |
| | ppm | 0 | 0 | 1 | 604 | 80 |
| | % von (4) | 0 | 0 | 0 | 100 | 100 |
| PN | kg/h | 0 | 0 | 0,01 | 0,26 | 0,28 |
| | ppm | 0 | 0 | 3 | 151 | 21 |
| | % von (4) | 0 | 0 | 3,7 | 96,3 | 100 |

**Tabelle 6: Bilanz Nitril-Nebenprodukte Beispiel 4 (Vergleichsbeispiel)**

| | | (7) | (13a) | (6) | (11) | (4) |
|---|---|---|---|---|---|---|
| ACN | kg/h | 12,38 | 1,98 | 1,17 | 0 | 15,52 |
| | Gew.-% | 0,15 | 0,19 | 0,03 | 0 | 0,12 |
| | % von (4) | 79,7 | 12,8 | 7,5 | 0 | 100 |
| ACRN | kg/h | 0,430 | 0,02 | 0 | 0,61 | 1,06 |
| | ppm | 53 | 15 | 1 | 356 | 80 |
| | % von (4) | 40,6 | 1,9 | 0 | 57,5 | 100 |
| PN | kg/h | 0,23 | 0,04 | 0,01 | 0,0 | 0,28 |
| | ppm | 28 | 33 | 3 | 0 | 21 |
| | % von (4) | 82,1 | 14,3 | 3,6 | 0 | 100 |
| HCN | kg/h | 13,0 | 0,39 | 0,0 | 1.705 | 1.726 |
| | Gew.-% | - | - | - | 99,84 | 13,05 |
| | ppm | 1.597 | 378 | 0 | - | - |

**Tabelle 7: Bilanz Nitril-Nebenprodukte Beispiel 5 (erfindungsgemäß)**

| | | (7) | (13c) | (6) | (11) | (4) |
|---|---|---|---|---|---|---|
| ACN | kg/h | 13,64 | 0,46 | 3,45 | 10,23 | 26,04 |
| | Gew.-% | 0,6002 | 0,1124 | 0,051 | 0,18 | 0,31 |
| | % von (4) | 52,4 | 1,8 | 13,2 | 39,3 | 100 |
| ACRN | kg/h | 0 | 0 | 0 | 0,09 | 0,1 |
| | ppm | 0 | 0 | 0 | 16 | 12 |
| | % von (4) | 0 | 0 | 0 | 90 | 100 |
| PN | kg/h | 0,04 | 0 | 0 | 0 | 0,04 |
| | ppm | 17 | 0 | 0 | 0 | 5 |
| | % von (4) | 100 | 0 | 0 | 0 | 100 |

**Tabelle 8: Bilanz Nitril-Nebenprodukte Beispiel 6 (Vergleichsbeispiel)**

| | | (7) | (13c) | (6) | (11) | (4) |
|---|---|---|---|---|---|---|
| ACN | kg/h | 0,24 | 0 | 3,45 | 24,06 | 26,04 |
| | Gew.-% | 0,01 | 0 | 0,051 | 0,01 | 0,31 |
| | % von (4) | 0,9 | 0 | 13,2 | 92,4 | 100 |
| ACRN | kg/h | 0 | 0 | 0 | 0,1 | 0,1 |
| | ppm | 0 | 0 | 0 | 16 | 12 |
| | % von (4) | 0 | 0 | 0 | 100 | 100 |
| PN | kg/h | 0 | 0 | 0 | 0,04 | 0,04 |
| | ppm | 0 | 0 | 0 | 5 | 5 |
| | % von (4) | 0 | 0 | 0 | 100 | 100 |

**Tabelle 9: Bilanz Nitril-Nebenprodukte Beispiel 7 (Vergleichsbeispiel)**

| | | (7) | (13c) | (6) | (11) | (4) |
|---|---|---|---|---|---|---|
| ACN | kg/h | 23,48 | 0,83 | 3,45 | 0 | 26,04 |
| | Gew.-% | 1,03 | 0,19 | 0,051 | 0 | 0,31 |
| | % von (4) | 90,2 | 3,2 | 13,2 | 0 | 100 |
| ACRN | kg/h | 0 | 0 | 0 | 0,09 | 0,1 |
| | ppm | 0 | 0 | 0 | 16 | 12 |
| | % von (4) | 0 | 0 | 0 | 90 | 100 |
| PN | kg/h | 0,04 | 0 | 0 | 0 | 0,04 |
| | ppm | 17 | 0 | 0 | 0 | 5 |
| | % von (4) | 100 | 0 | 0 | 0 | 100 |

Die Konzentration der Nitrile ACN, ACRN und PN, in Gew.-% bezogen auf die wässrige Phase in Abhängigkeit der theoretischen Stufe der Destillationskolonne (E) wird in den Figuren 5 bis 8 gezeigt.

In den Vergleichsbeispielen 4 und 7, bei denen sich ein hoher Anteil der Nitril-Nebenprodukte im Sumpf der Kolonne (E) befindet und eine Ausschleusung der Nitril-Nebenprodukte über (13a), (6) und (7) erfolgt, lagen Wassergehalt und Reinheit des erhaltenen HCN-Reinstroms (11) im gewünschten Bereich, allerdings kam es bei dieser Reaktionsführung zu einer Anreicherung von Nitril-Nebenprodukten in der Destillationskolonne (E), nämlich in einem Konzentrationsbereich von 7 bis 70 Gew.-% Acrylnitril (ACN) über 18 theoretische Stufen (Stufen 7-25) (Variante A, Beispiel 4, Fig. 6b) oder in einem Konzentrationsbereich von 7 bis 35 Gew.% ACN über 3 theoretische Stufen (Stufen 4-7) (Variante B, Beispiel 7, Fig. 8b). Bei Überschreitung der Grenzkonzentration von 7 Gew.-% ACN zerfallen ternäre Mischungen aus Wasser, Blausäure und ACN unter Bildung einer Acrylnitril-angereicherten Phase (siehe Fig. 9). Stabilisatoren, die üblicherweise zur Verhinderung der Polymerisation zugegeben werden, verbleiben in der wässrigen Phase und können daher die Polymerisation von Nitrilen in der überwiegend organischen Phase nicht mehr wirksam verhindern. Es kommt verstärkt zur Bildung von Ablagerungen. Es zeigte sich, dass in diesen Fällen eine ausreichende Ausschleusung der Nitrile, z.B. über Strom (13a), nicht möglich ist.

In Figur 6b (Beispiel 4) liegt die Konzentrationen an ACRN im Bereich der Stufen 10-20 bei fast 70 %, während bei den Beispielen 1-3 (Figuren 5a, 5b, und 6a) die Konzentration ACRN deutlich unter 10 Gew.-% liegt. In Vergleichsbeispiel 4 kam es daher vermehrt zu einer unerwünschten Polymerisation und störenden Ablagerungen im Bereich der der Stufen 10-20.

In Figur 8b (Beispiel 7) liegt die Konzentrationen an ACRN im Bereich der Stufen 4-7 bei deutlich über 10 Gew.-% und bei bis zu 35 Gew.-%, während bei den Beispielen 5-6 (Figuren 7 und 8a) die Konzentration ACRN unter 0,35 Gew.-% liegt. In Vergleichsbeispiel 7 kam es daher vermehrt zu einer unerwünschten Polymerisation und störenden Ablagerungen im Bereich der der Stufen 4-7.

Zudem zeigt sich, dass der Verlust von HCN über das ausgeschleuste Armgas (7) im Falle von Vergleichsbeispiel 4 deutlich höher ist als im erfindungsgemäßen Beispiel 2.

In den Vergleichsbeispielen 3 (Variante A, Fig. 6a) und 6 (Variante B, Fig. 8a), bei denen sich ein hoher Anteil an Nitril-Nebenprodukten im Kopfstrom der Kolonne (E) befindet und eine Ausschleusung der Nitril-Nebenprodukte hauptsächlich über den HCN-Reinstrom (11) und z.T. über den wässrigen Ammoniumsulfat-haltigen Strom (6) erfolgt, tritt das Problem der Ablagerungen in der Destillationskolone nicht auf. Allerdings verbleibt hier ein hoher Anteil an Nitril-Nebenprodukten im HCN-Reinprodukt (11). Gewünscht ist ein HCN-Reinprodukt (11) mit einem Wassergehalt ≤ 1,0 Gew.-%, bevorzugt ≤ 0,7 Gew.-%, besonders bevorzugt ≤ 0,6 Gew.-% und einem Anteil an C2-C10-Nitrile (in der Summe) ≤ 5.000 ppm, bevorzugt ≤4.000 ppm, besonders bevorzugt ≤ 3.000 ppm. Das HCN-Reinprodukt (11) weist im Fall der Vergleichsbeispiele 3 und 6 eine unzureichende Reinheit, nämlich einen zu hohen Wassergehalt von 1,1 Gew.% (Variante B, Beispiel 6) bzw. einen zu hohen Anteil an Nitrilen von 8955 ppm (Variante A, Beispiel 3) auf.

Es wurde überraschend gefunden, dass durch eine spezielle, ausgewogenen Ausschleusung der Nitril-Nebenprodukte über das HCN-Reinprodukt (11) sowie über einen oder mehrere der Stoffströme (13a), (6) und/oder (7), sowohl ein HCN-Produkt mit gewünschter Reinheit erhalten werden kann, als auch die Polymerisation von Nitril-Nebenprodukte in der Destillationskolonne verhindert werden kann. Wie in den Beispielen 1, 2 (Variante A) und 5 (Variante B) verdeutlicht, kann sowohl ein HCN-Reinprodukt mit einem Wassergehalt ≤ 0,6 Gew.-% bzw. mit einem Nitril-Gehalt ≤ 5.000 ppm erhalten werden, als auch die Acrylnitril-Konzentration in der Destillationskolonne (E) niedrig gehalten werden (Beispiel 1, Variante A, Fig. 5a: max. 6.5 Gew.% Acrynitril; Beispiel 2, Variante A, Fig. 5b: max. 2.6 Gew.% Acrynitril; Beispiel 5, Variante B, Fig. 7: max. 0.3 Gew.% Acrynitril). Eine Polymerisation oder die Bildung von Ablagerungen trat in diesen Beispielen nicht auf.

## Patentansprüche

1. Verfahren zur Herstellung von Blausäure (HCN), umfassend die Schritte
i. Umsetzung einer Methan-Quelle, einer Ammoniak-Quelle und optional einer Sauerstoff-Quelle in einem Reaktor in einem Reaktionsteil I unter Verwendung eines platinhaltigen Kontaktes, wobei ein HCN-haltiges Prozessgas erhalten wird, welches Nitril-Nebenprodukte, insbesondere ausgewählt aus C2-C10 Nitrilen, insbesondere ausgewählt aus Acetonitril, Acrylnitril und Propionitril, enthält;
ii. Absorption von nicht umgesetztem Ammoniak in einem Aufarbeitungsteil II, wobei das HCN-haltige Prozessgas in mindestens einer ersten Absorptionsvorrichtung, mit einem sauren, wässrigen Absorptionsmittel behandelt wird, wobei ein Ammoniak-abgereichertes Prozessgas und eine wässrige Ammoniumsulfat-haltige Lösung erhalten werden;
iii. Absorption von HCN im Aufarbeitungsteil II, wobei das Ammoniak-abgereicherte Prozessgas in mindestens einer zweiten Absorptionsvorrichtung mit einem wässrigen Absorptionsmittel behandelt wird, wobei eine erste wässrige HCN-haltige Lösung und ein HCN-abgereichertes Armgas erhalten werden;
iv. Erwärmen der ersten wässrigen HCN-haltigen Lösung im Aufarbeitungsteil II in mindestens einer Wärmeaustauschapparatur, wobei eine zweite wässrige HCN-haltige Lösung erhalten wird;
v. Destillation der zweiten wässrigen HCN-haltigen Lösung im Aufarbeitungsteil II in mindestens einer Destillationsvorrichtung, wobei ein HCN-Reinstrom und ein Nitril-haltiger, wässriger Sumpfstrom erhalten werden;
wobei
a. die Methan-Quelle mindestens 87,0 Vol.-% Methan; 0,2 bis 8,0 Vol.-% C2-C10 Alkane und optional bis zu 5,0 Vol.-% Inerte, beispielsweise ausgewählt aus Stickstoff, Wasserstoff und Kohlenoxiden, jeweils bezogen auf die gesamte Methan-Quelle, enthält;
b. die im HCN-haltigen Prozessgas enthaltenen Nitril-Nebenprodukte, insbesondere ausgewählt aus C2-C10 Nitrilen, insbesondere ausgewählt aus Acetonitril, Acrylnitril und Propionitril, durch die folgenden Schritte aus dem Verfahren ausgeschleust werden,
indem die wässrige Ammoniumsulfat-haltige Lösung, welche in Schritt ii) erhalten wird, und welche einen pH-Wert im Bereich von 2 bis 5 aufweist, vollständig oder teilweise aus dem Verfahren ausgeschleust wird;
indem der Nitril-haltige, wässrige Sumpfstrom, welcher in Schritt v) erhalten wird, zumindest teilweise aus dem Verfahren ausgeschleust wird und/oder zumindest teilweise in die erste Absorptionsvorrichtung in Schritt ii) zurückgeführt wird;
indem der HCN-Reinstrom, welcher in Schritt v) erhalten wird, 500 bis 5.000 ppm, bevorzugt 600 bis 4.500 ppm, besonders bevorzugt 800 bis 4.500 ppm, C2-C10-Nitrile, insbesondere ausgewählt aus Acetonitril, Acrylnitril und Propionitril und 10 bis 10.000 ppm, bevorzugt 100 bis 7.000 ppm, besonders bevorzugt 200 bis 6.000 ppm, Wasser, jeweils bezogen auf den gesamten HCN-Reinstrom, enthält;
und indem der Nitril-haltige, wässrige Sumpfstrom, welcher in Schritt v) erhalten wird, 10 bis 1.800 ppm, bevorzugt 50 bis 1.600 ppm, besonders bevorzugt 100 bis 1.500 ppm, C2-C10-Nitrile, insbesondere ausgewählt aus Acetonitril, Acrylnitril und Propionitril, bezogen auf den gesamten wässrige Sumpfstrom, enthält.

2. Verfahren nach Anspruch 1, **dadurch gekennzeichnet, dass** die Methan-Quelle 87,0 bis 99,8 Vol.-% Methan; 0,2 bis 5,0 Vol.-% Ethan; 0,0 bis 3,0 Vol.-% Propan und 0,0 bis 5,0 Vol.% Inerte, beispielsweise ausgewählt aus Stickstoff, Wasserstoff und Kohlenoxiden, jeweils bezogen auf die gesamte Methan-Quelle, enthält.

3. Verfahren nach Anspruch 1 oder 2, **dadurch gekennzeichnet, dass** ein Teil des Nitril-haltigen, wässrigen Sumpfstroms, welcher in Schritt v) erhalten wird, zum Erwärmen der ersten wässrigen HCN-haltigen Lösung in der Wärmeaustauschapparatur in Schritt iv) verwendet wird.

4. Verfahren nach einem der Ansprüche 1 bis 3, **dadurch gekennzeichnet, dass** das HCN-haltige Prozessgas, welches in Schritt i) erhalten wird, 6 bis 30 Vol.-% HCN und 10 bis 10.000 ppm, bevorzugt 20 bis 8.000 ppm, besonders bevorzugt 100 bis 6.000 ppm, Nitril-Nebenprodukte, bevorzugt ausgewählt aus C2-C10-Nitrilen, insbesondere ausgewählt aus Acetonitril, Acrylnitril und Propionitril, jeweils bezogen auf das gesamte Prozessgas, enthält.

5. Verfahren nach einem der Ansprüche 1 bis 4, **dadurch gekennzeichnet, dass** in Schritt iii) das Ammoniak-abgereicherte Prozessgas bei 0° bis 20°C und bei einem Druck von 1 bara bis 10 bara mit dem wässrigen Absorptionsmittel behandelt wird, wobei die erhaltene erste wässrige HCN-haltige Lösung 1 bis 50 Gew.-% HCN, 50 bis 98.9999 Gew.-% Wasser und 1 bis 2.000 ppm an Nitril-Nebenprodukten, insbesondere ausgewählt aus C2-C10 Nitrile, enthält, jeweils bezogen auf die gesamte erste wässrige HCN-haltige Lösung.

6. Verfahren nach einem der Ansprüche 1 bis 5, **dadurch gekennzeichnet, dass** in Schritt v) die Destillation der zweiten wässrigen HCN-haltigen Lösung bei einem Druck von 500 mbar bis 3000 mbar absolut in mindestens einer Destillationskolonne, durchgeführt wird, wobei die gemittelte Temperatur im mittleren Bereich der Destillationskolonne im Bereich von 45 bis 97 °C, bevorzugt 60 bis 96 °C, liegt.

7. Verfahren nach einem der Ansprüche 1 bis 6, **dadurch gekennzeichnet, dass** der HCN-Reinstrom, welcher in Schritt v) erhalten wird, 500 bis 5.000 ppm, bevorzugt 500 bis 4.000 ppm Acetonitril; 0 bis 1.000 ppm, bevorzugt 10 bis 800 ppm C3-Nitrile, insbesondere ausgewählt aus Acrylnitril und Propionitril, als Nitril-Nebenprodukt und 100 bis 7.000 ppm, bevorzugt 200 bis 6.000 ppm, Wasser, jeweils bezogen auf den gesamten HCN-Reinstrom, enthält.

8. Verfahren nach einem der Ansprüche 1 bis 7, **dadurch gekennzeichnet, dass** der Nitril-haltige, wässrige Sumpfstrom, welcher in Schritt v) erhalten wird, mehr als 99,0 Gew.-% Wasser und 10 bis 1.800 ppm, bevorzugt 50 bis 1.600 ppm, besonders bevorzugt 100 bis 1.500 ppm Acetonitril und 0 bis 1.000 ppm, bevorzugt 0 bis 800 ppm C3-Nitrile, insbesondere Acrylnitril und Propionitril, als Nitril-Nebenprodukt, jeweils bezogen auf den gesamten Nitril-haltigen, wässrigen Sumpfstrom, enthält.

9. Verfahren nach einem der Ansprüche 1 bis 8, **dadurch gekennzeichnet, dass** der Nitril-haltige, wässrige Sumpfstrom, welcher in Schritt v) erhalten wird, zumindest teilweise aus dem Verfahren ausgeschleust wird, wobei der ausgeschleuste Strom 10 bis 1.800 ppm, bevorzugt 50 bis 1.600 ppm, besonders bevorzugt 100 bis 1.500 ppm, C2-C10-Nitrile, insbesondere ausgewählt aus Acetonitril, Acrylnitril und Propionitril, bezogen auf den gesamten ausgeschleusten Strom, enthält.

10. Verfahren nach einem der Ansprüche 1 bis 9, **dadurch gekennzeichnet, dass** der Nitril-haltige, wässrige Sumpfstrom, welcher in Schritt v) erhalten wird, zumindest teilweise in die erste Absorptionsvorrichtung in Schritt ii) zurückgeführt wird, wobei der in Schritt ii) zurückgeführt Strom, 10 bis 1.800 ppm, bevorzugt 50 bis 1.600 ppm, besonders bevorzugt 100 bis 1.500 ppm, C2-C10-Nitrile, insbesondere ausgewählt aus Acetonitril, Acrylnitril und Propionitril, bezogen auf den gesamten zurückgeführt Strom, enthält.

11. Verfahren nach einem der Ansprüche 1 bis 10, **dadurch gekennzeichnet, dass** der Nitril-haltige, wässrige Sumpfstrom, welcher in Schritt v) erhalten wird, zumindest teilweise in mindestens einer Stripp-Kolonne mit einem Stripp-Gas behandelt wird, wobei die enthaltenen Nitril-Nebenprodukte, insbesondere ausgewählt aus C2-C10 Nitrilen, zumindest teilweise entfernt werden, und wobei ein gereinigter wässriger Strom erhalten wird, der zumindest teilweise aus dem Verfahren ausgeschleust wird und/oder zumindest teilweise in die erste Absorptionsvorrichtung (B) in Schritt ii) zurückgeführt wird (18c).

12. Verfahren nach einem der Ansprüche 1 bis 11, **dadurch gekennzeichnet, dass** die wässrige Ammoniumsulfat-haltige Lösung, welche in Schritt ii) erhalten wird, 10 bis 1.000 ppm, bevorzugt 10 bis 700 ppm, bezogen auf die gesamte wässrige Ammoniumsulfat-haltige Lösung, C2-C10-Nitrile, insbesondere ausgewählt aus Acetonitril, Acrylnitril und Propionitril, enthält, wobei die wässrige Ammoniumsulfat-haltige Lösung vollständig aus dem Verfahren ausgeschleust wird.

13. Verfahren nach einem der Ansprüche 1 bis 12, **dadurch gekennzeichnet, dass** die wässrige Ammoniumsulfat-haltige Lösung, welche in Schritt ii) erhalten wird, einer Aufreinigung zur Entfernung von Blausäure und/oder Nitrilen unterzogen wird, und optional Ammoniumsulfat aus der gereinigten wässrigen Lösung durch Kristallisation isoliert wird.

14. Verfahren zur Herstellung von Methacrylsäure und/oder Alkylmethacrylaten, insbesondere Methylmethacrylat (MMA), umfassend die folgenden Schritte:
xi. Herstellung von Blausäure in einem Verfahren gemäß einem der Ansprüche 1 bis 13, wobei ein HCN-Reinstrom erhalten wird;
xii. Umsetzung des HCN-Reinstroms erhalten in Schritt xi) und Aceton in Gegenwart eines basischen Katalysators in einer ersten Reaktionsstufe, wobei eine erste Reaktionsmischung enthaltend Acetoncyanhydrin (ACH) erhalten wird;
xiii. Aufarbeitung der ersten Reaktionsmischung, enthaltend Acetoncyanhydrin (ACH);
xiv. Umsetzung von Acetoncyanhydrin und Schwefelsäure in einem oder mehreren Reaktoren I in einer zweiten Reaktionsstufe bei einer Amidierungstemperatur, bevorzugt im Bereich von 85°C bis 130°, wobei eine zweite Reaktionsmischung, enthaltend Sulfoxyisobuttersäureamid und Methacrylamid, erhalten wird;
xv. Konvertieren der zweiten Reaktionsmischung, umfassend Erhitzen auf eine Konvertierungstemperatur, bevorzugt im Bereich von 130°C bis 200°C, in einem oder mehreren Reaktoren II in einer dritten Reaktionsstufe, wobei eine dritte Reaktionsmischung, enthaltend überwiegend Methacrylamid und Schwefelsäure, erhalten wird;
xvi. Umsetzung der dritten Reaktionsmischung mit Wasser und optional Alkohol, bevorzugt Wasser und optional Methanol, in einem oder mehreren Reaktoren III in einer vierten Reaktionsstufe, wobei eine vierte Reaktionsmischung, enthaltend Methacrylsäure und/oder Alkylmethacrylat, bevorzugt Methylmethacrylat, erhalten wird;
xvii. optional Aufarbeitung der vierten Reaktionsmischung zur Isolierung von Methacrylsäure und / oder Alkylmethacrylaten, insbesondere Methylmethacrylat.

## Claims

1. Process for production of hydrogen cyanide (HCN), comprising the steps of:
i. reacting a methane source, an ammonia source and optionally an oxygen source in a reactor in a reaction portion I using a platinum-containing solid catalyst to obtain an HCN-containing process gas containing nitrile byproducts, in particular selected from C2-C10 nitriles, in particular selected from acetonitrile, acrylonitrile and propionitrile;
ii. absorbing unconverted ammonia in a workup portion II, wherein the HCN-containing process gas is treated with an acidic, aqueous absorption medium in at least one first absorption apparatus to obtain an ammonia-depleted process gas and an aqueous ammonium sulfate-containing solution;
iii. absorbing HCN in the workup portion II, wherein the ammonia-depleted process gas is treated with an aqueous absorption medium in at least one second absorption apparatus to obtain a first aqueous HCN-containing solution and an HCN-depleted lean gas;
iv. heating the first aqueous HCN-containing solution in the workup portion II in at least one heat exchange apparatus to obtain a second aqueous HCN-containing solution;
v. distilling the second aqueous HCN-containing solution in the workup portion II in at least one distillation apparatus to obtain a pure HCN stream and a nitrile-containing, aqueous bottoms stream;
wherein
a. the methane source contains at least 87.0% by volume of methane; 0.2% to 8.0% by volume of C2-C10 alkanes and optionally up to 5.0% by volume of inerts, for example selected from nitrogen, hydrogen and carbon oxides, in each case based on the total methane source;
b. the nitrile byproducts present in the HCN-containing process gas, in particular selected from C2-C10 nitriles, in particular selected from acetonitrile, acrylonitrile and propionitrile, are discharged from the process via the following steps:
the aqueous ammonium sulfate-containing solution obtained in step ii) and having a pH in the range from 2 to 5 is completely or partially discharged from the process;
the nitrile-containing, aqueous bottoms stream obtained in step v) is at least partially discharged from the process and/or at least partially recycled into the first absorption apparatus in step ii);
the pure HCN stream obtained in step v) contains 500 to 5000 ppm, preferably 600 to 4500 ppm, particularly preferably 800 to 4500 ppm, of C2-C10 nitriles, in particular selected from acetonitrile, acrylonitrile and propionitrile, and 10 to 10 000 ppm, preferably 100 to 7000 ppm, particularly preferably 200 to 6000 ppm, of water, in each case based on the total pure HCN stream;
and the nitrile-containing, aqueous bottoms stream obtained in step v) contains 10 to 1800 ppm, preferably 50 to 1600 ppm, particularly preferably 100 to 1500 ppm, of C2-C10 nitriles, in particular selected from acetonitrile, acrylonitrile and propionitrile, based on the total aqueous bottoms stream.

2. Process according to Claim 1, **characterized in that** the methane source contains 87.0% to 99.8% by volume of methane; 0.2% to 5.0% by volume of ethane; 0.0% to 3.0% by volume of propane and 0.0% to 5.0% by volume of inerts, for example selected from nitrogen, hydrogen and carbon oxides, in each case based on the total methane source.

3. Process according to Claim 1 or 2, **characterized in that** a portion of the nitrile-containing, aqueous bottoms stream obtained in step v) is used for heating the first aqueous HCN-containing solution in the heat exchange apparatus in step iv) .

4. Process according to any of Claims 1 to 3, **characterized in that** the HCN-containing process gas obtained in step i) contains 6% to 30% by volume of HCN and 10 to 10 000 ppm, preferably 20 to 8000 ppm, particularly preferably 100 to 6000 ppm, of nitrile byproducts, preferably selected from C2-C10 nitriles, in particular selected from acetonitrile, acrylonitrile and propionitrile, in each case based on the total process gas.

5. Process according to any of Claims 1 to 4, **characterized in that** in step iii) the ammonia-depleted process gas is treated with the aqueous absorption medium at 0°C to 20°C and at a pressure of 1 bara to 10 bara, wherein the obtained first aqueous HCN-containing solution contains 1% to 50% by weight of HCN, 50% to 98.9999% by weight of water and 1 to 2000 ppm of nitrile byproducts, in particular selected from C2-C10 nitriles, in each case based on the total first aqueous HCN-containing solution.

6. Process according to any of Claims 1 to 5, **characterized in that** in step v) the distillation of the second aqueous HCN-containing solution is performed in at least one distillation column at a pressure of 500 mbar to 3000 mbar absolute, wherein the average temperature in the middle region of the distillation column is preferably in the range from 45°C to 97°C, preferably 60°C to 96°C.

7. Process according to any of Claims 1 to 6, **characterized in that** the pure HCN stream obtained in step v) contains 500 to 5000 ppm, preferably 500 to 4000 ppm, of acetonitrile; 0 to 1000 ppm, preferably 10 to 800 ppm, of C3 nitriles, in particular selected from acrylonitrile and propionitrile, as nitrile byproduct and 100 to 7000 ppm, preferably 200 to 6000 ppm, of water, in each case based on the total pure HCN stream.

8. Process according to any of Claims 1 to 7, **characterized in that** the nitrile-containing, aqueous bottoms stream obtained in step v) contains more than 99.0% by weight of water and 10 to 1800 ppm, preferably 50 to 1600 ppm, particularly preferably 100 to 1500 ppm, of acetonitrile and 0 to 1000 ppm, preferably 0 to 800 ppm, of C3 nitriles, in particular acrylonitrile and propionitrile, as nitrile byproduct, in each case based on the total nitrile-containing, aqueous bottoms stream.

9. Process according to any of Claims 1 to 8, **characterized in that** the nitrile-containing, aqueous bottoms stream obtained in step v) is at least partially discharged from the process, wherein the discharged stream contains 10 to 1800 ppm, preferably 50 to 1600 ppm, particularly preferably 100 to 1500 ppm, of C2-C10 nitriles, in particular selected from acetonitrile, acrylonitrile and propionitrile, based on the total discharged stream.

10. Process according to any of Claims 1 to 9, **characterized in that** the nitrile-containing, aqueous bottoms stream obtained in step v) is at least partially recycled into the first absorption apparatus in step ii), wherein the stream recycled in step ii) contains 10 to 1800 ppm, preferably 50 to 1600 ppm, particularly preferably 100 to 1500 ppm, of C2-C10 nitriles, in particular selected from acetonitrile, acrylonitrile and propionitrile, based on the total recycled stream.

11. Process according to any of Claims 1 to 10, **characterized in that** the nitrile-containing, aqueous bottoms stream obtained in step v) is at least partially treated with a stripping gas in at least one stripping column, wherein the nitrile byproducts present, in particular selected from C2-C10 nitriles, are at least partially removed to obtain a purified aqueous stream which is at least partially discharged from the process and/or at least partially recycled into the first absorption apparatus (B) in step ii) (18c).

12. Process according to any of Claims 1 to 11, **characterized in that** the aqueous ammonium sulfate-containing solution obtained in step ii) contains 10 to 1000 ppm, preferably 10 to 700 ppm, based on the total aqueous ammonium sulfate-containing solution, of C2-C10 nitriles, in particular selected from acetonitrile, acrylonitrile and propionitrile, wherein the aqueous ammonium sulfate-containing solution is completely discharged from the process.

13. Process according to any of Claims 1 to 12, **characterized in that** the aqueous ammonium sulfate-containing solution obtained in step ii) is subjected to a purification for removing hydrogen cyanide and/or nitriles and ammonium sulfate is optionally isolated from the purified aqueous solution by crystallization.

14. Process for production of methacrylic acid and/or alkyl methacrylates, in particular methyl methacrylate (MMA), comprising the steps of:
xi. producing hydrogen cyanide in a process according to any of Claims 1 to 13 to obtain a pure HCN stream;
xii. reacting the pure HCN stream obtained in step xi) and acetone in the presence of a basic catalyst in a first reaction stage to obtain a first reaction mixture containing acetone cyanohydrin (ACH);
xiii. working up the first reaction mixture containing acetone cyanohydrin (ACH);
xiv. reacting acetone cyanohydrin and sulfuric acid in one or more reactors I in a second reaction stage at an amidation temperature, preferably in the range from 85°C to 130°C, to obtain a second reaction mixture containing sulfoxyisobutyramide and methacrylamide;
xv. converting the second reaction mixture, comprising heating to a conversion temperature, preferably in the range from 130°C to 200°C, in one or more reactors II in a third reaction stage to obtain a third reaction mixture containing predominantly methacrylamide and sulfuric acid;
xvi. reacting the third reaction mixture with water and optionally alcohol, preferably water and optionally methanol, in one or more reactors III in a fourth reaction stage to obtain a fourth reaction mixture containing methacrylic acid and/or alkyl methacrylate, preferably methyl methacrylate;
xvii. optionally working up the fourth reaction mixture to isolate methacrylic acid and/or alkyl methacrylates, in particular methyl methacrylate.

## Revendications

1. Procédé pour la préparation d'acide cyanhydrique (HCN), comprenant les étapes
i. transformation d'une source de méthane, d'une source d'ammoniac et éventuellement d'une source d'oxygène dans un réacteur, dans une partie de réacteur I avec utilisation d'un contact contenant du platine, un gaz de processus contenant du HCN étant obtenu qui contient des produits secondaires de type nitrile, en particulier choisis parmi les nitriles en C2-C10, en particulier choisis parmi l'acétonitrile, l'acrylonitrile et le propionitrile ;
ii. absorption d'ammoniac qui n'a pas réagi dans une partie de retraitement II, le gaz de processus contenant du HCN étant traité, dans au moins un premier dispositif d'absorption, par un absorbant aqueux acide, un gaz de processus appauvri en ammoniac et une solution aqueuse contenant du sulfate d'ammonium étant obtenus ;
iii. absorption de HCN dans la partie de retraitement II, le gaz de processus appauvri en ammoniac étant traité dans au moins un deuxième dispositif d'absorption par un absorbant aqueux, une première solution aqueuse contenant du HCN et un gaz pauvre appauvri en HCN étant obtenus ;
iv. chauffage de la première solution aqueuse contenant du HCN dans la partie de retraitement II dans au moins un appareil d'échange thermique, une deuxième solution aqueuse contenant du HCN étant obtenue ;
v. distillation de la deuxième solution aqueuse contenant du HCN dans la partie de retraitement II dans au moins un dispositif de distillation, un flux de HCN pur et un flux de fond aqueux contenant des nitriles étant obtenus ;
dans lequel
a. la source de méthane contient au moins 87,0% en volume de méthane ; 0,2 à 8,0% en volume d'alcanes en C2-C10 et éventuellement jusqu'à 5,0% en volume de substances inertes, par exemple choisies parmi l'azote, l'hydrogène et les oxydes de carbone, à chaque fois par rapport à la totalité de la source de méthane ;
b. les produits secondaires de type nitrile contenus dans le gaz de processus contenant du HCN, en particulier choisis parmi les nitriles en C2-C10, en particulier choisis parmi l'acétonitrile, l'acrylonitrile et le propionitrile, étant soutirés du procédé par les étapes suivantes,
la solution aqueuse contenant du sulfate d'ammonium, qui est obtenue dans l'étape ii) et qui présente un pH dans la plage de 2 à 5, étant soutirée complètement ou partiellement du procédé ;
le flux de fond aqueux contenant des nitriles, qui est obtenu dans l'étape v), étant soutiré au moins partiellement du procédé et/ou recyclé au moins partiellement dans le premier dispositif d'absorption dans l'étape ii) ;
le flux de HCN pur, qui est obtenu dans l'étape v), contenant 500 à 5000 ppm, de préférence 600 à 4500 ppm, de manière particulièrement préférée 800 à 4500 ppm, de nitriles en C2-C10, en particulier choisis parmi l'acétonitrile, l'acrylonitrile et le propionitrile et 10 à 10.000 ppm, de préférence 100 à 7000 ppm, de manière particulièrement préférée 200 à 6000 ppm, d'eau, à chaque fois par rapport à la totalité du flux de HCN pur ;
et le flux de fond aqueux contenant des nitriles, qui est obtenu dans l'étape v), contenant 10 à 1800 ppm, de préférence 50 à 1600 ppm, de manière particulièrement préférée 100 à 1500 ppm, de C2-C10-nitriles, en particulier choisis parmi l'acétonitrile, l'acrylonitrile et le propionitrile, par rapport à la totalité du flux de fond aqueux.

2. Procédé selon la revendication 1, **caractérisé en ce que** la source de méthane contient 87,0 à 99,8% en volume de méthane ; 0,2 à 5,0% en volume d'éthane ; 0,0 à 3,0% en volume de propane et 0,0 à 5,0% en volume de substances inertes, par exemples choisies parmi l'azote, l'hydrogène et les oxydes de carbone, chaque fois par rapport à la totalité de la source de méthane.

3. Procédé selon la revendication 1 ou 2, **caractérisé en ce qu'**une partie du flux de fond aqueux contenant des nitriles, qui est obtenu dans l'étape v), est utilisée pour le chauffage de la première solution aqueuse contenant du HCN dans l'appareil d'échange thermique dans l'étape iv).

4. Procédé selon l'une des revendications 1 à 3, **caractérisé en ce que** le gaz de processus contenant du HCN, qui est obtenu dans l'étape i), contient 6 à 30% en volume de HCN et 10 à 10.000 ppm, de préférence 20 à 8000 ppm, de manière particulièrement préférée 100 à 6000 ppm, de produits secondaires de type nitrile, de préférence choisis parmi les nitriles en C2-C10, en particulier choisis parmi l'acétonitrile, l'acrylonitrile et le propionitrile, à chaque fois par rapport à la totalité du gaz de processus.

5. Procédé selon l'une des revendications 1 à 4, **caractérisé en ce que** dans l'étape iii), le gaz de processus appauvri en ammoniac est traité à 0°C jusqu'à 20°C et à une pression de 1 bar abs. à 10 bars abs. par l'absorbant aqueux, la première solution aqueuse contenant du HCN obtenue contenant 1 à 50% en poids de HCN, 50 à 98,9999% en poids d'eau et 1 à 2000 ppm de produits secondaires de type nitrile, en particulier choisis parmi les nitriles en C2-C10, à chaque fois par rapport à la totalité de la première solution aqueuse contenant du HCN.

6. Procédé selon l'une des revendications 1 à 5, **caractérisé en ce que** dans l'étape v), la distillation de la deuxième solution aqueuse contenant du HCN est réalisée à une pression de 500 mbars à 3000 mbars absolus dans au moins une colonne de distillation, la température moyenne dans la zone centrale de la colonne de distillation se situant dans la plage de 45 à 97°C, de préférence de 60 à 96°C.

7. Procédé selon l'une des revendications 1 à 6, **caractérisé en ce que** le flux de HCN pur, qui est obtenu dans l'étape v), contient 500 à 5000 ppm, de préférence 500 à 4000 ppm d'acétonitrile ; 0 à 1000 ppm, de préférence 10 à 800 ppm de nitriles en C3, en particulier choisis parmi l'acrylonitrile et le propionitrile, en tant que produit secondaire de type nitrile et 100 à 7000 ppm, de préférence 200 à 6000 ppm, d'eau, à chaque fois par rapport à la totalité du flux de HCN pur.

8. Procédé selon l'une des revendications 1 à 7, **caractérisé en ce que** le flux de fond aqueux contenant des nitriles, qui est obtenu dans l'étape v), contient plus de 99,0% en poids d'eau et 10 à 1800 ppm, de préférence 50 à 1600 ppm, de manière particulièrement préférée 100 à 1500 ppm d'acétonitrile et 0 à 1000 ppm, de préférence 0 à 800 ppm de nitriles en C3, en particulier l'acrylonitrile et le propionitrile, en tant que produit secondaire de type nitrile, à chaque fois par rapport à la totalité du flux de fond aqueux contenant des nitriles.

9. Procédé selon l'une des revendications 1 à 8, **caractérisé en ce que** le flux de fond aqueux contenant des nitriles, qui est obtenu dans l'étape v), est soutiré au moins partiellement du procédé, le flux soutiré contenant 10 à 1800 ppm, de préférence 50 à 1600 ppm, de manière particulièrement préférée 100 à 1500 ppm, de nitriles en C2-C10, en particulier choisis parmi l'acétonitrile, l'acrylonitrile et le propionitrile, par rapport à la totalité du flux soutiré.

10. Procédé selon l'une des revendications 1 à 9, **caractérisé en ce que** le flux de fond aqueux contenant des nitriles, qui est obtenu dans l'étape v), est recyclé au moins partiellement dans le premier dispositif d'absorption dans l'étape ii), le flux recyclé dans l'étape ii) contenant 10 à 1800 ppm, de préférence 50 à 1600 ppm, de manière particulièrement préférée 100 à 1500 ppm, de nitriles en C2-C10, en particulier choisis parmi l'acétonitrile, l'acrylonitrile et le propionitrile, par rapport à la totalité du flux recyclé.

11. Procédé selon l'une des revendications 1 à 10, **caractérisé en ce que** le flux de fond aqueux contenant des nitriles, qui est obtenu dans l'étape v), est traité au moins partiellement dans au moins une colonne de rectification par un gaz de rectification, les produits secondaires de type nitrile contenus, en particulier choisis parmi les nitriles en C2-C10, étant au moins partiellement éliminés et un flux aqueux purifié étant obtenu, qui est soutiré au moins partiellement du procédé et/ou recyclé au moins partiellement dans le premier dispositif d'absorption (B) dans l'étape ii) (18c).

12. Procédé selon l'une des revendications 1 à 11, **caractérisé en ce que** la solution aqueuse contenant du sulfate d'ammonium, qui est obtenue dans l'étape ii), contient 10 à 1000 ppm, de préférence 10 à 700 ppm, par rapport à la totalité de la solution aqueuse contenant du sulfate d'ammonium, de nitriles en C2-C10, en particulier choisis parmi l'acétonitrile, l'acrylonitrile et le propionitrile, la solution aqueuse contenant du sulfate d'ammonium étant complètement soutirée du procédé.

13. Procédé selon l'une des revendications 1 à 12, **caractérisé en ce que** la solution aqueuse contenant du sulfate d'ammonium, qui est obtenue dans l'étape ii), est soumise à une purification pour l'élimination d'acide cyanhydrique et/ou de nitriles et le sulfate d'ammonium est éventuellement isolé à partir de la solution aqueuse purifiée par cristallisation.

14. Procédé de préparation d'acide méthacrylique et/ou de méthacrylates de méthyle, en particulier de méthacrylate de méthyle (MMA), comprenant les étapes suivantes :
xi. préparation d'acide cyanhydrique dans un procédé selon l'une des revendications 1 à 13, un flux de HCN pur étant obtenu ;
xii. transformation du flux de HCN pur obtenu dans l'étape xi) et d'acétone en présence d'un catalyseur basique dans un premier étage de réaction, un premier mélange réactionnel contenant de la cyanhydrine d'acétone (ACH) étant obtenu ;
xiii. retraitement du premier mélange réactionnel, contenant la cyanhydrine d'acétone (ACH) ;
xiv. transformation de cyanhydrine d'acétone et d'acide sulfurique dans un ou plusieurs réacteurs I dans un deuxième étage de réaction à une température d'amidation, de préférence dans la plage de 85°C à 130°C, un deuxième mélange réactionnel, contenant de l'amide de l'acide sulfoxyisobutyrique et du méthacrylamide, étant obtenu ;
xv. conversion du deuxième mélange réactionnel, comprenant le chauffage à une température de conversion, de préférence dans la plage de 130 à 200°C, dans un ou plusieurs réacteurs II dans un troisième étage de réaction, un troisième mélange réactionnel, contenant principalement du méthacrylamide et de l'acide sulfurique, étant obtenu ;
xvi. transformation du troisième mélange réactionnel avec de l'eau et éventuellement avec un alcool, de préférence avec de l'eau et éventuellement avec du méthanol, dans un ou plusieurs réacteurs III dans un quatrième étage de réaction, un quatrième mélange réactionnel, contenant de l'acide méthacrylique et/ou du méthacrylate d'alkyle, de préférence du méthacrylate de méthyle, étant obtenu ;
xvii. retraitement éventuel du quatrième mélange réactionnel pour l'isolement d'acide méthacrylique et/ou de méthacrylates d'alkyle, en particulier de méthacrylate de méthyle.
